(19) Europäisches Patentamt
European Patent Office
Office européen des brevets

(11) Veröffentlichungsnummer: 0 362 635 A1

# (12) EUROPÄISCHE PATENTANMELDUNG

(21) Anmeldenummer: 89117497.1

(51) Int. Cl.5: C07D 211/90 , C07D 401/12 , A61K 31/44

(22) Anmeldetag: 21.09.89

(30) Priorität: 05.10.88 DE 3833893

(43) Veröffentlichungstag der Anmeldung: 11.04.90 Patentblatt 90/15

(84) Benannte Vertragsstaaten: AT BE CH DE ES FR GB GR IT LI LU NL SE

(71) Anmelder: BAYER AG

D-5090 Leverkusen 1 Bayerwerk(DE)

(72) Erfinder: Stoltefuss, Jürgen, Dipl.-Ing.
Parkstrasse 20
D-5657 Haan 2(DE)
Erfinder: Schwenner, Eckhard, Dr.
Paul-Ehrlich-Strasse 29
D-5600 Wuppertal 1(DE)
Erfinder: Gross, Rainer, Prof. Dr.
Platzhoftrasse 23
D-5600 Wuppertal 1(DE)
Erfinder: Hebisch, Siegbert, Dr.
Richard-Seel-Weg 11
D-5600 Wuppertal 1(DE)
Erfinder: Schramm, Matthias, Dr.
Paffrather Strasse 38
D-5000 Köln 80(DE)
Erfinder: Bechem, Martin, Dr.
Obere Bergerheide 4
D-5600 Wuppertal 1(DE)
Erfinder: Hirth, Claudia, Dr.
Claudiusweg 9
D-5600 Wuppertal 1(DE)
Erfinder: Stasch, Johannes-Peter, Dr.
Schneewittchenweg 37
D-5600 Wuppertal 1(DE)

## (54) Verfahren von basischen Nitrophenyl-dihydropyridinamiden als Arzneimittel, neue Verbindungen und Verfahren zu ihrer Herstellung über neue Zwischenprodukte.

(57) Die vorliegende Erfindung betrifft die Verwendung von teilweise bekannten Nitro-phenyl-dihydropyridinamiden der allgemeinen Formel I

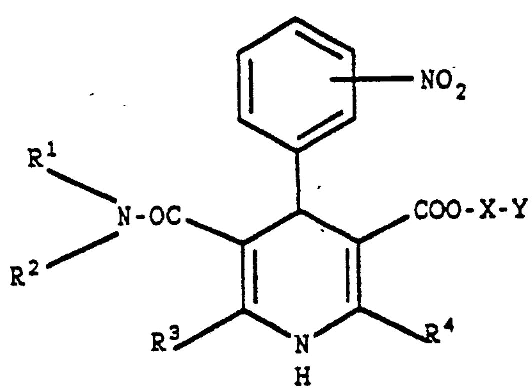

(I)

in welcher R¹ bis R⁴, X und Y die in der Beschreibung angegebene Bedeutung haben, als Arzneimittel, neue Wirkstoffe und Verfahren zu ihrer Herstellung über neue Zwischenprodukte, insbesondere die Verwendung als kreislaufbeeinflussende Arzneimittel.



Xerox Copy Centre

# Verwendung von basischen Nitro-phenyl-dihydropyridinamiden als Arzneimittel, neue Verbindungen und Verfahren zu ihrer Herstellung über neue Zwischenprodukte

Die vorliegende Erfindung betrifft die Verwendung von teilweise bekannten Nitro-phenyl-dihydropyridinamiden als Arzneimittel, neue Wirkstoffe und Verfahren zu ihrer Herstellung über neue Zwischenprodukte, insbesondere die Verwendung als kreislaufbeeinflussende Arzneimittel.

Es ist bereits bekannt, daß basische 4-Aryl-dihydropyridinamine als Zwischenprodukte zur Darstellung von 1,4-Dihydropyridin-hydroxyalkylaminen eingesetzt werden [vgl. EP-A 0 179 386].

In dieser Publikation werden Dihydropyridinderivate beschrieben, die in 3-Position eine Hydroxyalkylaminestergruppe tragen. Der Substituent X in 5-Position umfaßt in seiner allgemeinen Bedeutung auch Amidreste. Die Herstellung der dort beschriebenen Wirkstoffe erfolgt unter anderem über Dihydropyridinamine der allgemeinen Formel (II) die dort als Zwischenprodukte eingesetzt werden. Die allgemeine Definition dieser Zwischenprodukte der Formel (II) umfaßt unter anderem auch Dihydropyridinamide ohne jedoch einen konkreten Vertreter dieser Stoffklasse zu benennen. Diese Publikation enthält auch keinen Hinweis auf irgendeine pharmakologische Wirkung dieser dort eingesetzten Zwischenprodukte.

In der DE-OS 22 28 377 werden Dihydropyridin-Carbonsäureamide, mehrere Verfahren zu ihrer Herstellung und ihre Verwendung als kreislaufbeeinflussende Mittel beschrieben. Die dort genannten Verbindungen unterscheiden sich von den erfindungsgemäßen Wirkstoffen dadurch, daß die 3- und 5-Position des Dihydropyridinringes entweder beide gleichzeitig eine Amidgruppe tragen oder eine dieser Positionen eine Estergruppe trägt, die jedoch ihrerseits keine basischen Gruppen mehr enthält. Im Gegensatz dazu enthält der Esterrest der erfindungsgemäß zu verwendenden Verbindungen immer einen basischen Rest (siehe Definition X und Y).

Auch in der EP-A 0 220 653 werden Dihydropyridinderivate beschrieben, die in 3-Position eine Amidgruppe und in 5-Position eine Estergruppe enthalten. Auch in diesem Falle trägt Estergruppe keinen basischen Rest mehr. Diese bekannten Monoamide besitzen ebenfalls Kreislaufwirkungen.

Es wurde gefunden, daß die 4-Nitrophenyl-dihydropyridinamide der allgemeinen Formel (I)

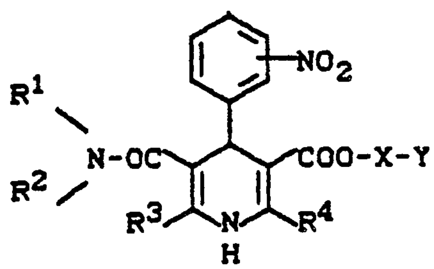

(I)

in welcher

$R^1$ und $R^2$ gleich oder verschieden sind und

- Wasserstoff bedeuten, oder
- für Cycloalkyl mit 3 bis 8 Kohlenstoffatomen stehen, oder
- für geradkettiges oder verzweigtes Alkyl oder Alkenyl, mit jeweils bis zu 12 Kohlenstoffatomen stehen, die gegebenenfalls substituiert sind durch Halogen, Hydroxy, Alkoxy mit bis zu 8 Kohlenstoffatomen, Alkylthio mit bis zu 8 Kohlenstoffatomen, Alkylcarbonyl mit bis zu 8 Kohlenstoffatomen, Carboxy oder Alkoxycarbonyl mit bis zu 8 Kohlenstoffatomen oder durch Phenyl, das seinerseits durch Halogen, Nitro, Cyano, Trifluormethyl, Trifluormethoxy, Alkyl mit bis zu 4 Kohlenstoffatomen oder Alkoxy mit bis zu 4 Kohlenstoffatomen oder durch eine Gruppe $-NR^5R^6$ substituiert sein kann,

worin

$R^5$ und $R^6$ gleich oder verschieden sind und Wasserstoff, Alkyl mit bis zu 8 Kohlenstoffatomen, Aryl oder Aralkyl bedeuten,

- für Phenyl stehen, das bis zu 3-fach gleich oder verschieden durch Nitro, Cyano, Halogen, Alkyl mit bis zu 6 Kohlenstoffatomen, Alkoxy mit bis zu 6 Kohlenstoffatomen, Alkylthio mit bis zu 6 Kohlenstoffatomen, Trifluormethyl, Trifluormethoxy, Difluormethoxy, Trifluormethylthio, Amino, Alkylamino mit bis zu 8 Kohlenstoffatomen je Alkylgruppe, Dialkylamino mit bis zu 8 Kohlenstoffatomen je Alkylgruppe, Acetylamino oder Benzoylamino substituiert sein kann, oder

$R^1$ und $R^2$ gemeinsam einen 5- bis 7-gliedrigen gesättigten oder ungesättigten, heterocyclischen Ring

bilden, der als zusätzliches Heteroatom ein Sauerstoffatom, ein Schwefelatom, ein Stickstoffatom, oder einen Rest N-$R^7$ enthalten kann,

worin

$R^7$ - Wasserstoff, $C_1$-$C_6$-Alkyl, Aryl oder Aralkyl bedeuten kann

und

$R^3$ und $R^4$ gleich oder verschieden sind und

- für geradkettiges, verzweigtes oder cyclisches Alkyl mit bis zu 8 Kohlenstoffatomen stehen, das gegebenenfalls durch Hydroxy, Cyano, Phenyl oder Halogen substituiert ist

und

X - für einen geradkettigen, verzweigten oder cyclischen, gesättigten oder ungesättigten Kohlenwasserstoffrest mit bis zu 12 Kohlenstoffatomen steht, der gegebenenfalls durch ein Sauerstoff-, Schwefel- oder Stickstoffatom, oder eine Gruppe N-$R^8$ unterbrochen ist,

worin

$R^8$ - Wasserstoff bedeutet, oder

- für Alkyl mit bis zu 6 Kohlenstoffatomen steht, das gegebenenfalls substituiert sein kann durch Halogen, Hydroxy, Acetoxy, Carboxy, $C_1$-$C_8$-Alkoxycarbonyl oder Phenyl, das seinerseits substituiert sein kann durch Halogen, $C_1$-$C_6$-Alkyl, $C_1$-$C_6$-Alkoxy, Halogenmethyl, Halogenmethoxy oder Cyano,

und

Y - Pyridyl bedeutet, oder

- für eine Gruppe der Formel -$NR^9R^{10}$ steht,

worin

$R^9$ und $R^{10}$ gleich oder verschieden sind und

- Wasserstoff bedeuten, oder

- für geradkettiges oder verzweigtes Alkyl, Alkenyl oder Alkinyl mit jeweils bis zu 12 Kohlenstoffatomen stehen, das substituiert sein kann durch Halogen, Hydroxy, Alkoxy mit bis zu 8 Kohlenstoffatomen, Cyano, Trifluormethyl, Alkylthio mit bis zu 8 Kohlenstoffatomen, Alkylcarbonyl mit bis zu 8 Kohlenstoffatomen, Alkoxycarbonyl mit bis zu 8 Kohlenstoffatomen oder durch Phenyl, welches seinerseits durch Nitro, Phenyl, Cyano, Trifluormethyl, Trifluormethoxy, Alkyl mit bis zu 4 Kohlenstoffatomen, Halogen oder Alkoxy mit bis zu 4 Kohlenstoffatomen substituiert sein kann, oder

- für Cycloalkyl mit 3 bis 8 Kohlenstoffatomen stehen, oder

- für Aryl mit 6 bis 10 Kohlenstoffatomen stehen, das bis zu 3-fach gleich oder verschieden durch Nitro, Cyano, Halogen, Alkyl mit bis zu 6 Kohlenstoffatomen, Alkoxy mit bis zu 6 Kohlenstoffatomen Alkylthio mit bis zu 6 Kohlenstoffatomen, Carbamoyl, Dialkylcarbamoyl mit jeweils bis zu 6 Kohlenstoffatomen je Alkylgruppe, Trifluormethyl, Trifluormethoxy, Difluormethoxy, Trifluormethylthio, Amino, Alkylamino mit bis zu 8 Kohlenstoffatomen, Dialkylamino mit jeweils bis zu 8 Kohlenstoffatomen je Alkylgruppe substituiert sein kann, oder

$R^9$ und $R^{10}$ zusammen einen 5- bis 7-gliedrigen, gesättigten oder ungesättigten, heterocyclischen Ring bilden, der ein Sauerstoffatom, ein Schewefelatom oder ein zusätzliches Stickstoffatom enthalten kann, das gegebenenfalls durch einen Rest $R^{11}$ substituiert ist,

worin

$R^{11}$ - für Wasserstoff steht, oder

- für eine geradkettige oder verzweigte, gesättigte oder ungesättigte Alkylen gruppe mit bis zu 10 Kohlenstoffatomen steht, die gegebenenfalls durch Phenyl substituiert ist, welches seinerseits durch Halogen, $C_1$-$C_4$-Alkyl, $C_1$-$C_4$-Alkoxy, Nitro oder Halogenalkyl mit bis zu 4 Kohlenstoffatomen substituiert sein kann, oder

- für Phenyl steht, das gegebenenfalls durch Halogen, Cyano, Nitro, Alkyl mit bis zu 4 Kohlenstoffatomen, Alkoxy mit bis zu 4 Kohlenstoffatomen oder Halogenalkyl mit bis zu 4 Kohlenstoffatomen substituiert ist,

neben der erwarteten Calcium-antagonistischen Aktivität überraschenderweise auch eine starke antiarrhythmische Wirkung zeigen und den Salz- und Flüssigkeitshaushalt beeinflussen und somit geeignet sind zur Verwendung bei der Bekämpfung der Hypertonie und der Herzinsuffizienz.

Auch die physiologisch unbedenklichen Säureadditionssalze der Verbindungen der allgemeinen Formel (I) sowie die Racemformen, die Antipoden und die Diastereomerengemische sind bevorzugt geeignet für diese neuartige Verwendung.

Bevorzugt verwendet werden Verbindungen der allgemeinen Formel (I),

in welcher

$R^1$ und $R^2$ gleich oder verschieden sind und

- Wasserstoff bedeuten, oder

- für Cycloalkyl mit 3 bis 8 Kohlenstoffatomen stehen, oder

- für geradkettiges oder verzweigtes Alkyl oder Alkenyl, mit jeweils bis zu 10 Kohlenstoffatomen stehen, die gegebenenfalls substituiert sind durch Fluor, Chlor, Hydroxy, $C_1$-$C_6$-Alkoxy, $C_1$-$C_6$-Alkylthio, $C_1$-$C_6$-Alkylcarbonyl, $C_1$-$C_6$-Alkoxycarbonyl, Carboxy oder durch Phenyl, welches seinerseits durch Halogen, Nitro, Cyano, Trifluormethyl, Trifluormethoxy, Methyl, Methoxy oder durch eine Gruppe der Formel $-NR^5R^6$ substituiert sein kann,
worin
$R^5$ und $R^6$ gleich oder verschieden sind und
- für Wasserstoff, $C_1$-$C_6$-Alkyl, Benzyl, Phenethyl oder Phenyl stehen,
- für Phenyl stehen, das gegebenenfalls bis zu 2-fach gleich oder verschieden substituiert ist durch Fluor, Chlor, $C_1$-$C_4$-Alkyl, $C_1$-$C_4$-Alkoxy, $C_1$-$C_4$-Alkylthio, Trifluormethyl, Trifluormethoxy, Difluormethoxy, oder
$R^1$ und $R^2$ gemeinsam für Pyridyl, Pyrrolidyl, Piperidyl, Morpholinyl, oder
- für gegebenenfalls durch $C_1$-$C_4$-Alkyl, Benzyl oder Phenethyl substituiertes Piperazinyl stehen,
$R^3$ und $R^4$ gleich oder verschieden sind und
- für geradkettiges oder verzweigtes Alkyl mit bis zu 6 Kohlenstoffatomen stehen, das gegebenenfalls durch Fluor, Chlor, Phenyl oder Hydroxy substituiert ist,
X - für einen geradkettigen, verzweigten oder cyclischen, gesättigten oder ungesättigten Kohlenwasserstoffrest mit bis zu 10 Kohlenstoffatomen steht, der gegebenenfalls durch ein Sauerstoff-, ein Schwefel- oder ein Stickstoffatom, oder durch einen Rest $N-R^8$ unterbrochen ist,
worin
$R^8$ - Wasserstoff bedeutet, oder
- für $C_1$-$C_4$-Alkyl steht, das gegebenenfalls substituiert ist durch Fluor, Chlor, Hydroxy, Carboxy oder durch Phenyl, welches seinerseits durch Fluor, Chlor, $C_1$-$C_4$-Alkyl, $C_1$-$C_4$-Alkoxy, Trifluormethyl oder Trifluormethoxy substituiert sein kann,
und
Y - Pyridyl bedeutet, oder
- für eine Gruppe der Formel $-NR^9R^{10}$ steht,
worin
$R^9$ und $R^{10}$ gleich oder verschieden sind und
- Wasserstoff bedeuten, oder
- für geradkettiges oder verzweigtes Alkyl oder Alkenyl mit jeweils bis zu 8 Kohlenstoffatomen stehen, das substituiert sein kann durch Fluor, Chlor, Hydroxy, $C_1$-$C_4$-Alkoxy, $C_1$-$C_4$-Alkylthio, $C_1$-$C_4$-Alkylcarbonyl, Carboxy oder $C_1$-$C_6$-Alkoxycarbonyl, oder durch Phenyl, welches seinerseits durch Trifluormethyl, $C_1$-$C_2$-Alkyl, Fluor, Chlor oder $C_1$-$C_2$-Alkoxy substituiert sein kann, oder
- für Cycloalkyl mit 3 bis 8 Kohlenstoffatomen stehen, oder
- für Phenyl stehen, das bis zu 2-fach gleich oder verschieden durch Nitro, Cyano, Fluor, Chlor, Brom, $C_1$-$C_4$-Alkyl, $C_1$-$C_4$-Alkoxy, $C_1$-$C_4$-Alkylthio, Trifluormethyl, Trifluormethoxy, Difluormethoxy, Trifluormethylthio, Amino, Alkylamino mit bis zu 4 Kohlenstoffatomen substituiert sein kann, oder
$R^9$ und $R^{10}$ zusammen einen 5- bis 7-gliedrigen gesättigten oder ungesättigten, heterocyclischen Ring bilden, der ein Sauerstoff- oder Schwefelatom oder ein zusätzliches Stickstoffatom enthalten kann, welches gegebenenfalls durch einen Rest $R^{11}$ substituiert ist,
worin
$R^{11}$ - für Wasserstoff steht, oder
- für eine geradkettige oder verzweigte, gesättigte oder ungesättigte Alkylgruppe mit bis zu 4 Kohlenstoffatomen steht, die gegebenenfalls durch Phenyl substituiert ist, welches seinerseits durch Fluor, Chlor, $C_1$-$C_2$-Alkyl, $C_1$-$C_2$-Alkoxy oder $C_1$-$C_2$-Halogenalkyl substituiert sein kann, oder
- für Phenyl steht, das gegebenenfalls durch Fluor, Chlor, Cyano, $C_1$-$C_2$-Alkyl, $C_1$-$C_2$-Alkoxy oder $C_1$-$C_2$-Halogenalkyl substituiert ist
und deren physiologisch unbedenklichen Salze zur Bekämpfung der Hypertonie und der Herzinsuffizienz.

Besonders bevorzugt werden Verbindungen der allgemeinen Formel (I) verwendet,
in welcher
$R^1$ und $R^2$ gleich oder verschieden sind und
- für Wasserstoff, Cyclopropyl, Cyclopentyl oder Cyclohexyl stehen, oder
- für geradkettiges oder verzweigtes Alkyl oder Alkenyl mit bis zu 8 Kohlenstoffatomen stehen, das gegebenenfalls substituiert ist durch Fluor, Chlor, Hydroxy, $C_1$-$C_4$-Alkoxy, $C_1$-$C_4$-Alkylthio, Carboxy, $C_1$-$C_4$-Alkoxycarbonyl oder durch Phenyl, das seinerseits durch Chlor, Fluor, Methyl oder Methoxy substituiert sein kann, oder
- für Phenyl stehen, das substituiert sein kann durch Fluor, Chlor, Methyl, Ethyl, Methoxy, Trifluormethyl, Trifluormethoxy,

$R^3$ und $R^4$
- für Methyl stehen
und
X - für einen geradkettigen, verzweigten oder cyclischen, gesättigten oder ungesättigten Kohlenwasserstoffrest mit bis zu 8 Kohlenstffatomen steht, der gegebenenfalls durch ein Sauerstoff-, Schwefel-oder Stickstoffatom, oder durch einen Rest $-N-R^8$ unterbrochen ist,
worin
$R^8$ - Wasserstoff bedeutet, oder
- für Methyl, Ethyl, Benzyl, Phenethyl oder Phenyl steht, das gegebenenfalls Fluor, Chlor, Methyl, Methoxy, Trifluormethyl oder Trifluormethoxy substituiert ist,
Y - für Pyridyl steht, oder
- für eine Gruppe der Formel $-NR^9R^{10}$ steht,
worin
$R^9$ und $R^{10}$ gleich oder verschieden sind und
- Wasserstoff bedeuten, oder
- für geradkettiges oder verzweigtes Alkyl oder Alkenyl mit bis zu 4 Kohlenstoffatomen stehen, das substituiert sein kann durch Fluor, Chlor, Hydroxy, $C_1$-$C_4$-Alkoxycarbonyl oder durch Phenyl, das seinerseits durch Fluor, Chlor, Trifluormethyl, Methyl oder Methoxy substituiert sein kann, oder
- für Cyclopropyl, Cyclopentyl oder Cyclohexyl stehen, oder
- für Phenyl stehen, das bis zu 2-fach gleich oder verschieden durch Fluor, Chlor, Methyl, Ethyl, Methoxy, Ethoxy, Methylthio, Trifluormethyl, Trifluormethoxy, Difluormethoxy, Amino, $C_1$-$C_2$-Alkylamino oder $C_1$-$C_2$-Dialkylamino substituiert sein kann, oder
$R^9$ und $R^{10}$ gemeinsam einen 5- bis 7-gliedrigen gesättigten Ring bilden, der ein Sauerstoff-, Schwefel- oder ein zusätzliches Stickstoffatom enthalten kann, das gegebenenfalls durch einen Rest $R^{11}$ substituiert ist,
worin
$R^{11}$ - für Wasserstoff, $C_1$-$C_4$-Alkyl, Benzyl oder Phenethyl steht, oder
- für Phenyl steht, das durch Fluor, Chlor, Methyl oder Methoxy substituiert sein kann
und deren physiologisch unbedenklichen Salze bei der Bekämpfung der Hypertonie und der Herzinsuffizienz.

Die erfindungsgemäßen Verbindungen zeigen ein nicht vorhersehbares, wertvolles, pharmakologisches Wirkspektrum. Sie beeinflussen die Kontraktionskraft des Herzens, den Tonus der glatten Muskulatur sowie den Elektrolyt- und Flüssigkeitshaushalt.

Sie können deshalb für die Herstellung von Arzneimitteln zur Behandlung des pathologisch veränderten Blutdrucks und der Herzinsuffizienz, sowie von Coronartherapeutika eingesetzt werden.

Darüberhinaus können sie bei der Herstellung von Arzneimitteln zur Behandlung von Herzrhythmusstörungen, Niereninsuffizienz, Leberzirrhose, Aszites, Lungenödem, Hirnödem, Schwangerschaftödem, Glaukom oder Diabetes mellitus eingesetzt werden.

Die Erfindung betrifft außerdem neue 4-Nitrophenyl-dihydropyridinamide der allgemeinen Formel (Ia)

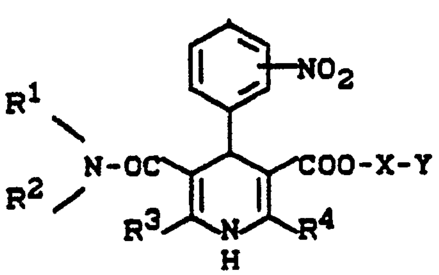

(Ia)

in welcher
$R^1$ und $R^2$ gleich oder verschieden sind und
- Wasserstoff bedeuten, oder
- für Cycloalkyl mit 3 bis 8 Kohlenstoffatomen stehen, oder
- für geradkettiges oder verzweigtes Alkyl oder Alkenyl, mit jeweils bis zu 12 Kohlenstoffatomen stehen, die gegebenenfalls substituiert sind durch Halogen, Hydroxy, Alkoxy mit bis zu 8 Kohlenstoffatomen, Alkylthio mit bis zu 8 Kohlenstoffatomen, Alkylcarbonyl mit bis zu 8 Kohlenstoffatomen, Carboxy oder Alkoxycarbonyl mit bis zu 8 Kohlenstoffatomen oder durch Phenyl, das seinserseits durch Halogen, Nitro, Cyano,

Trifluormethyl, Trifluormethoxy, Alkyl mit bis zu 4 Kohlenstoffatomen oder Alkoxy mit bis zu 4 Kohlenstoffatomen oder durch eine Gruppe $-NR^5R^6$ substituiert sein kann,
worin
$R^5$ und $R^6$ gleich oder verschieden sind und Wasserstoff, Alkyl mit bis zu 8 Kohlenstoffatomen bedeuten,
- für Phenyl stehen, das bis zu 3-fach gleich oder verschieden durch Nitro, Cyano, Halogen, Alkyl mit bis zu 6 Kohlenstoffatomen, Alkoxy mit bis zu 6 Kohlenstoffatomen, Alkylthio mit bis zu 6 Kohlenstoffatomen, Trifluormethyl, Trifluormethoxy, Difluormethoxy, Trifluormethylthio, Amino, Alkylamino mit bis zu 8 Kohlenstoffatomen, Dialkylamino mit bis zu 8 Kohlenstoffatomen je Alkylgruppe, Acetylamino oder Benzoylamino substituiert sein kann, oder
$R^1$ und $R^2$ gemeinsam einen 5- bis 7-gliedrigen gesättigten oder ungesättigten, heterocyclischen Ring bilden, der als zusätzliches Heteroatom ein Sauerstoffatom, ein Schwefelatom, ein Stickstoffatom, oder einen Rest $N-R^7$ enthalten kann,
worin
$R^7$ - Wasserstoff, $C_1$-$C_6$-Alkyl, Aryl oder Aralkyl bedeuten kann
und
$R^3$ und $R^4$ gleich oder verschieden sind und
- für geradkettiges, verzweigtes oder cyclisches Alkyl mit bis zu 8 Kohlenstoffatomen stehen, das gegebenenfalls durch Hydroxy, Cyano, Phenyl oder Halogen substituiert ist
und
X - für einen geradkettigen, verzweigten oder cyclischen, gesättigten oder ungesättigten Kohlenwasserstoffrest mit bis zu 12 Kohlenstoffatomen steht, der gegebenenfalls durch ein Sauerstoff-, Schwefel- oder Stickstoffatom, oder eine Gruppe $N-R^8$ unterbrochen ist,
worin
$R^8$ - Wasserstoff bedeutet, oder
- für Alkyl mit bis zu 6 Kohlenstoffatomen steht, das gegebenenfalls substituiert sein kann durch Halogen, Hydroxy, Acetoxy, Carboxy, $C_1$-$C_8$-Alkoxycarbonyl oder Phenyl, das seinerseits substituiert sein kann durch Halogen, $C_1$-$C_6$-Alkyl, $C_1$-$C_6$-Alkoxy, Halogenmethyl, Halogenmethoxy oder Cyano,
und
Y- Pyridyl bedeutet, oder
- für eine Guppe der Formel $-NR^9R^{10}$ steht,
worin
$R^9$ und $R^{10}$ gleich oder verschieden sind und
- für Wasserstoff oder geradkettiges oder verzweigtes Alkyl, Alkenyl oder Alkinyl mit jeweils bis zu 12 Kohlenstoffatomen stehen, das substituiert sein kann durch Halogen, Hydroxy, Alkoxy mit bis zu 8 Kohlenstoffatomen, Cyano, Trifluormethyl, Alkylthio mit bis zu 8 Kohlenstoffatomen, Alkylcarbonyl mit bis zu 8 Kohlenstoffatomen, Alkoxycarbonyl mit bis zu 8 Kohlenstoffatomen oder durch Phenyl, welches seinerseits durch Nitro, Phenyl, Cyano, Trifluormethyl, Trifluormethoxy, Alkyl mit bis zu 4 Kohlenstoffatomen, Halogen, oder Alkoxy mit bis zu 4 Kohlenstoffatomen substituiert sein kann, oder
- für Cycloalkyl mit 3 bis 8 Kohlenstoffatomen stehen, oder
- für Aryl mit 6 bis 10 Kohlenstoffatomen stehen, das bis zu 3-fach gleich oder verschieden durch Nitro, Cyano, Halogen, Alkyl mit bis zu 6 Kohlenstoffatomen, Alkoxy mit bis zu 6 Kohlenstoffatomen, Alkylthio mit bis zu 6 Kohlenstoffatomen, Carbamoyl, Dialkylcarbamoyl mit jeweils bis zu 6 Kohlenstoffatomen je Alkylgruppe, Trifluormethyl, Trifluormethoxy, Difluormethoxy, Trifluormethylthio, Amino, Alkylamino mit bis zu 8 Kohlenstoffatomen, Dialkylamino mit jeweils bis zu 8 Kohlenstoffatomen je Alkylgruppe substituiert sein kann, oder
$R^9$ und $R^{10}$ zusammen einen 5- bis 7-gliedrigen, gesättigten oder ungesättigten, heterocyclischen Ring bilden, der ein Sauerstoffatom, ein Schwefelatom oder ein zusätzliches Stickstoffatom enthalten kann, das gegebenenfalls durch einen Rest $R^{11}$ substituiert ist,
worin
$R^{11}$ - für Wasserstoff steht, oder
- für eine geradkettige oder verzweigte, gesättigte oder ungesättigte Alkylengruppe mit bis zu 10 Kohlenstoffatomen steht, die gegebenenfalls durch Phenyl substituiert ist, welches seinerseits durch Halogen, $C_1$-$C_4$-Alkyl, $C_1$-$C_4$-Alkoxy, Nitro oder Halogenalkyl mit bis zu 4 Kohlenstoffatomen substituiert sein kann, oder
- für Phenyl steht, das gegebenenfalls durch Halogen, Cyano, Nitro, Alkyl mit bis zu 4 Kohlenstoffatomen, Alkoxy mit bis zu 4 Kohlenstoffatomen oder Halogenalkyl mit bis zu 4 Kohlenstoffatomen substituiert ist,
und deren physiologisch unbedenklichen Salze.

Die erfindungsgemäßen Verbindungen existieren in stereoisomeren Formen, die sich entweder wie Bild und Spiegelbild (Enantiomere) oder die sich nicht wie Bild und Spiegelbild (Diastereomere) verhalten. Die

Erfindung betrifft sowohl die Antipoden als auch die Racemformen sowie die Diastereomerengemische. Die Racemformen lassen sich ebenso wie die Diastereomeren in bekannter Weise in die steroisomer einheitlichen Bestandteile trennen (vgl. E.L. Eliel, Stereochemistry of Carbon Compounds, McGraw Hill, 1962).

Physiologisch unbedenkliche Salze können Salze der erfindungsgemäßen Verbindungen mit anorganischen oder organischen Säuren sein. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfel säure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Bevorzugt sind Verbindungen der allgemeinen Formel (Ia),
in welcher
$R^1$ und $R^2$ gleich oder verschieden sind und
- Wasserstoff bedeuten, oder
- für Cycloalkyl mit 3 bis 8 Kohlenstoffatomen stehen, oder
- für geradkettiges oder verzweigtes Alkyl oder Alkenyl, mit jeweils bis zu 10 Kohlenstoffatomen stehen, die gegebenenfalls substituiert sind durch Fluor, Chlor, Hydroxy, $C_1$-$C_6$-Alkoxy, $C_1$-$C_6$-Alkylthio, $C_1$-$C_6$-Alkylcarbonyl, $C_1$-$C_6$-Alkoxycarbonyl, Carboxy oder durch Phenyl, welches seinerseits durch Halogen, Nitro, Cyano, Trifluormethyl, Trifluormethoxy, Methyl, Methoxy oder durch eine Gruppe der Formel $-NR^5R^6$ substituiert sein kann,
worin
$R^5$ und $R^6$ gleich oder verschieden sind und
- für Wasserstoff, $C_1$-$C_6$-Alkyl, Benzyl, Phenethyl oder Phenyl stehen,
- für Phenyl stehen, das gegebenenfalls bis zu 2-fach gleich oder verschieden substituiert ist durch Fluor, Chlor, $C_1$-$C_4$-Alkyl, $C_1$-$C_4$-Alkoxy, $C_1$-$C_4$-Alkylthio, Trifluormethyl, Trifluormethoxy, Difluormethoxy, oder
$R^1$ und $R^2$ gemeinsam für Pyridyl, Pyrrolidyl, Piperidyl, Morpholinyl, oder
- für gegebenenfalls durch $C_1$-$C_4$-Alkyl, Benzyl oder Phenethyl substituiertes Piperazinyl stehen,
$R^3$ und $R^4$ gleich oder verschieden sind und
- für geradkettiges oder verzweigtes Alkyl mit bis zu 6 Kohlenstoffatomen stehen, das gegebenenfalls durch Fluor, Chlor, Phenyl oder Hydroxy substituiert ist,
X - für einen geradkettigen, verzweigten oder cyclischen, gesättigten oder ungesättigten Kohlenwasserstoffrest mit bis zu 10 Kohlenstoffatomen steht, der gegebenenfalls durch ein Sauerstoff-, ein Schwefel- oder ein Stickstoffatom, oder durch einen Rest $N$-$R^8$ unterbrochen ist,
worin
$R^8$ - Wasserstoff bedeutet, oder
- für $C_1$-$C_4$-Alkyl steht, das gegebenenfalls substituiert ist durch Fluor, Chlor, Hydroxy, Carboxy oder durch Phenyl, welches seinerseits durch Fluor, Chlor, $C_1$-$C_4$-Alkyl, $C_1$-$C_4$-Alkoxy, Trifluormethyl oder Trifluormethoxy substituiert sein kann,
und
Y - Pyridyl bedeutet, oder
- für eine Gruppe der Formel $-NR^9R^{10}$ steht,
worin
$R^9$ und $R^{10}$ gleich oder verschieden sind und
- für Wasserstoff oder geradkettiges oder verzweigtes Alkyl oder Alkenyl mit jeweils bis zu 8 Kohlenstoffatomen stehen, das substituiert sein kann durch Fluor, Chlor, Hydroxy, $C_1$-$C_4$-Alkoxy, $C_1$-$C_4$-Alkylthio, $C_1$-$C_4$-Alkylcarbonyl, Carboxy oder $C_1$-$C_6$-Alkoxycarbonyl, oder durch Phenyl, welches seinerseits durch Trifluormethyl $C_1$-$C_2$-Alkyl, Fluor, Chlor oder $C_1$-$C_2$-Alkoxy substituiert sein kann, oder
- für Cycloalkyl mit 3 bis 8 Kohlenstoffatomen stehen, oder
- für Phenyl stehen, das bis zu 2-faoh gleich oder verschieden durch Nitro, Cyano, Fluor, Chlor, Brom, $C_1$-$C_4$-Alkyl, $C_1$-$C_4$-Alkoxy, $C_1$-$C_4$-Alkylthio, Trifluormethyl, Trifluormethoxy, Difluormethoxy, Trifluormethylthio, Amino, Alkylamino mit bis zu 4 Kohlenstoffatomen substituiert sein kann, oder
$R^9$ und $R^{10}$ zusammen einen 5- bis 7-gliedrigen gesättigten oder ungesättigten, heterocyclischen Ring bilden, der ein Sauerstoff- oder Schwefelatom oder ein zusätzliches Stickstoffatom enthalten kann, welches gegebenenfalls durch einen Rest $R^{11}$ substituiert ist,
worin
$R^{11}$ - für Wasserstoff steht, oder
- für eine geradkettige oder verzweigte, gesättigte oder ungesättigte Alkylgruppe mit bis zu 4 Kohlenstoffatomen steht, die gegebenenfalls durch Phenyl substituiert ist, welches seinerseits durch Fluor, Chlor, $C_1$-$C_2$-Alkyl, $C_1$-$C_2$-Alkoxy oder $C_1$-$C_2$-Halogenalkyl substituiert sein kann, oder

- für Phenyl steht, das gegebenenfalls durch Fluor, Chlor, Cyano, $C_1$-$C_2$-Alkyl, $C_1$-$C_2$-Alkoxy oder $C_1$-$C_2$-Halogenalkyl substituiert ist
und deren physiologisch unbedenklichen Salze.

Besonders bevorzugt sind Verbindungen der allgemeinen Formel (Ia),
in welcher
$R^1$ und $R^2$ gleich oder verschieden sind und
- für Wasserstoff, Cyclopropyl, Cyclopentyl oder Cyclohexyl stehen, oder
- für geradkettiges oder verzweigtes Alkyl oder Alkenyl mit bis zu 8 Kohlenstoffatomen stehen, das gegebenenfalls substituiert ist durch Fluor, Chlor, Hydroxy, $C_1$-$C_4$-Alkoxy, $C_1$-$C_4$-Alkylthio, Carboxy, $C_1$-$C_4$-Alkoxycarbonyl oder durch Phenyl, das seinerseits durch Chlor, Fluor, Methyl oder Methoxy substituiert sein kann, oder
- für Phenyl stehen, das substituiert sein kann durch Fluor, Chlor, Methyl, Ethyl, Methoxy, Trifluormethyl, Trifluormethoxy,
$R^3$ und $R^4$
- für Methyl stehen
und
X - für einen geradkettigen, verzweigten oder cyclischen, gesättigten oder ungesättigten Kohlenwasserstoffrest mit bis zu 8 Kohlenstoffatomen steht, der gegebenenfalls durch ein Sauerstoff-, Schwefel-oder Stickstoffatom, oder durch einen Rest N-$R^8$ unterbrochen ist,
worin
$R^8$ - Wasserstoff bedeutet, oder
- für Methyl, Ethyl, Benzyl, Phenethyl oder Phenyl steht, das gegebenenfalls durch Fluor, Chlor, Methyl, Methoxy, Trifluormethyl oder Trifluormethoxy substituiert ist,
Y - für Pyridyl steht, oder
- für eine Gruppe der Formel -$NR^9R^{10}$ steht,
worin
$R^9$ und $R^{10}$ gleich oder verschieden sind und
- für Wasserstoff oder geradkettiges oder verzweigtes Alkyl oder Alkenyl mit jeweils bis zu 4 Kohlenstoffatomen stehen, das substituiert sein kann durch Fluor, Chlor, Hydroxy, $C_1$-$C_4$-Alkoxycarbonyl oder durch Phenyl, das seinerseits durch Fluor, Chlor, Trifluormethyl, Methyl oder Methoxy substituiert sein kann, oder
- für Cyclopropyl, Cyclopentyl oder Cyclohexyl stehen, oder
- für Phenyl stehen, das bis zu 2-fach gleich oder verschieden durch Fluor, Chlor, Methyl, Ethyl, Methoxy, Ethoxy, Methylthio, Trifluormethyl, Trifluormethoxy, Difluormethoxy, Amino, $C_1$-$C_2$-Alkylamino oder $C_1$-$C_2$-Dialkylamino substituiert sein kann, oder
$R^9$ und $R^{10}$ gemeinsam einen 5- bis 7-gliedrigen gesättigten Ring bilden, der ein Sauerstoff-, Schwefel- oder ein zusätzliches Stickstoffatom enthalten kann, das gegebenenfalls durch einen Rest $R^{11}$ substituiert ist,
worin
$R^{11}$ - für Wasserstoff, $C_1$-$C_4$-Alkyl, Benzyl oder Phenethyl steht, oder
- für Phenyl steht, das durch Fluor, Chlor, Methyl oder Methoxy substituiert sein kann
und deren physiologisch unbedenklichen Salze.

Ganz besonders bevorzugt sind Verbindungen der allgemeinen Formel (Ia) und (I) in welcher
$R^1$ und $R^2$ gleich oder verschieden sind und jeweils für
- Wasserstoff, Alkyl mit 1-4 C-Atomen, oder
- für Cyclopropyl oder Cyclopentyl stehen,
$R^3$ und $R^4$ für Methyl stehen,
X für eine geradkettige oder verzweigte Alkylkette mit bis zu 4 Kohlenstoffatomen steht, und
Y für Pyridyl oder Morpholino steht oder
für eine Gruppe der Formel -$NR^9R^{10}$ steht,
worin $R^9$ und $R^{10}$ gleich oder verschieden sind und jeweils Wasserstoff, Methyl, Ethyl oder Benzyl bedeuten,
und ihre physiologisch unbedenklichen Salze.

Auch die neuen erfindungsgemäßen Verbindungen der Formel (Ia) zeigen ein nicht vorhersehbares, wertvolles, pharmakologisches Wirkspektrum. Sie beeinflussen die Kontraktionskraft des Herzens, den Tonus der glatten Muskulatur sowie den Elektrolyt- und Flüssigkeitshaushalt.

Sie können deshalb in Arzneimitteln zur Behandlung des pathologisch veränderten Blutdrucks und der Herzinsuffizienz, sowie als Koronartherapeutika eingesetzt werden.

Darüberhinaus können sie zur Behandlung von Herzrhythmusstörungen, Niereninsuffizienz, Leberzirrhose, Aszites, Lungenödem, Hirnödem, Schwangerschaftödem, Glaukom oder Diabetes mellitus eingesetzt werden.

Die erfindungsgemäßen Verbindungen der allgemeinen Formel (I) bzw. (Ia)

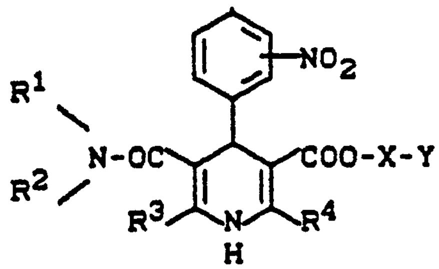

(I)

in welcher

$R^1$, $R^2$, $R^3$, $R^4$, X und Y die oben angegebene Bedeutung haben,

erhält man, indem man

[A] Aldehyde der allgemeinen Formel (II)

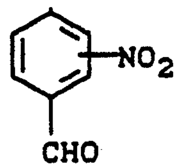

(II)

und β-Ketocarbonsäureester der allgemeinen Formel (III)

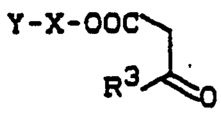

(III)

in welcher

$R^3$, X und Y die oben angegebene Bedeutung haben,

gegebenenfalls nach Isolierung der hieraus entstehenden Ylidenverbindung der allgemeinen Formel (IV)

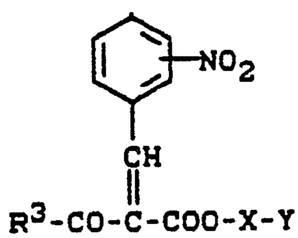

(IV)

in welcher

$R^3$, X und Y die oben angegebene Bedeutung haben,

mit β-Ketocarbonsäureamiden der allgemeinen Formel (V)

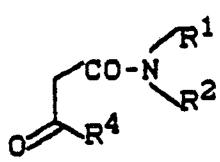

(V)

in welcher
$R^1$, $R^2$ und $R^4$ die oben angegebene Bedeutung haben,
und Ammoniak bzw. direkt mit den hieraus hergestellten β-Aminocrotonsäureamiden der allgemeinen Formel (VI)

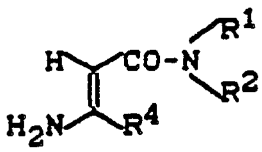

(VI)

in welcher
$R^1$, $R^2$ und $R^4$ die oben angegebene Bedeutung haben,
umsetzt,
oder indem man
[B] Aldehyde der allgemeinen Formel (II) und β-Ketocarbonsäureamiden der allgemeinen Formel (V) bzw. deren Ylidenverbindungen der allgemeinen Formel (VII)

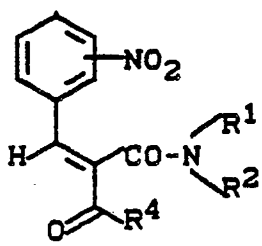

(VII)

in welcher
$R^1$, $R^2$ und $R^4$ die oben angegebene Bedeutung haben,
mit β-Ketocarbonsäureestern der allgemeinen Formel (III) und Ammoniak bzw. direkt mit den hieraus hergestellten Aminocrotonsäureestern der allgemeinen Formel (VIII)

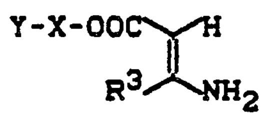

(VIII)

in welcher
$R^3$, X und Y die oben angegebene Bedeutung haben,
umsetzt,
oder man erhält Verbindungen der allgemeinen Formel (I),
in welcher
$R^1$, $R^2$, $R^3$, $R^4$ und X die oben angegebene Bedeutung haben,
Y für eine Gruppe der Formel $NR^9R^{10}$ steht und $R^9$ und/oder $R^{10}$ reaktionsfähigen Wasserstoff bedeuten, zweckmäßigerweise indem man
[C] Benzylidenverbindungen der allgemeinen Formel (VII) zunächst mit Verbindungen der allgemeinen Formel (VIIIa)

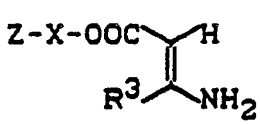

(VIIIa)

in welcher

$R^3$ und X die angegebene Bedeutung haben,
Z - für eine acylierte Aminoschutzgruppe, bevorzugt für tert.-Butoxycarbonylamino oder Phthalimid steht, zu Verbindungen der allgemeinen Formel (IX)

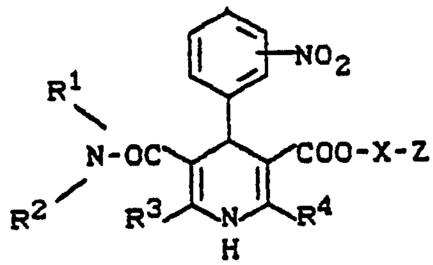
(IX)

in welcher
$R^1$, $R^2$, $R^3$, $R^4$ und X die oben angegebene Bedeutung haben,
und
Z - für die Gruppe

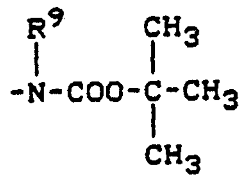
oder
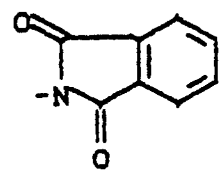
steht,

in einem weiteren Schritt nach bekannten Methoden deblockiert und die erhaltenen Zwischenverbindungen der Formel (IXa),

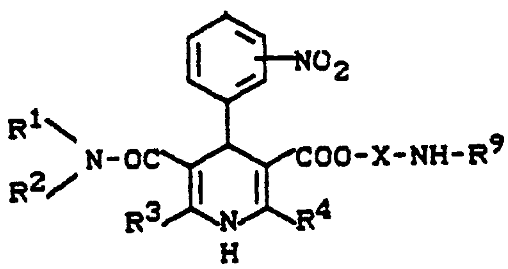
(IXa)

in welcher
$R^1$, $R^2$, $R^3$, $R^4$, X und $R^9$ die oben angegebene Bedeutung haben,
zu Verbindungen der allgemeinen Formel (I),
in welcher
Y - für die Gruppe $NR^9R^{10}$ steht, worin $R^9$ und $R^{10}$ die oben angebene Bedeutung haben, mit der Maßgabe, daß $R^9$ und/oder $R^{10}$ verschieden von Wasserstoff sind,
weiter umsetzt,
oder indem man
[D] Dihydropyridinmonocarbonsäuren der allgemeinen Formel (Xa) oder (Xb)

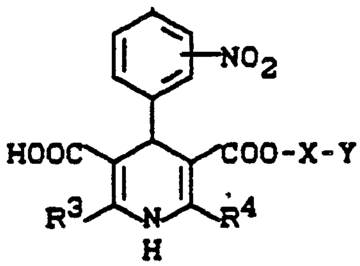

(Xa)

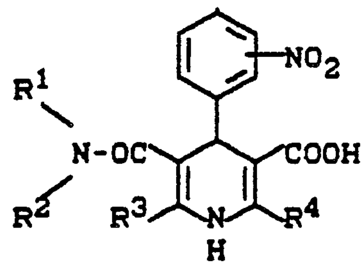

(Xb)

in welchen
$R^1$, $R^2$, $R^3$, $R^4$, X und Y die oben angegebene Bedeutung haben,
gegebenenfalls über reaktive Säurederivate mit Aminen der allgemeinen Formel (XIa)

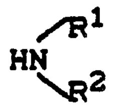

(XIa)

in welcher
$R^1$ und $R^2$ die oben angegebene Bedeutung haben,
bzw. mit Verbindungen der Formel (XIb)
HO-X-Y (XIb)
in welcher
X und Y die oben angegebene Bedeutung haben,
umsetzt, wobei jeweils nur (Xa) mit (XIa) und (Xb) mit (XIb) umgesetzt wird.

Als reaktive Säurederivate seien beispielsweise genannt: aktivierte Ester, Hydroxysuccinimidester, Säureimidazolide, Säurehalogenide, gemischte Anhydride oder die Umsetzung in Anwesenheit von Cyclohexylcarbodiimid.

Je nach Art der verwendeten Ausgangsstoffe können die Synthesevarianten für die erfindungsgemäßen Verbindungen durch folgende Formelschemata wiedergegeben werden:

Variante A:

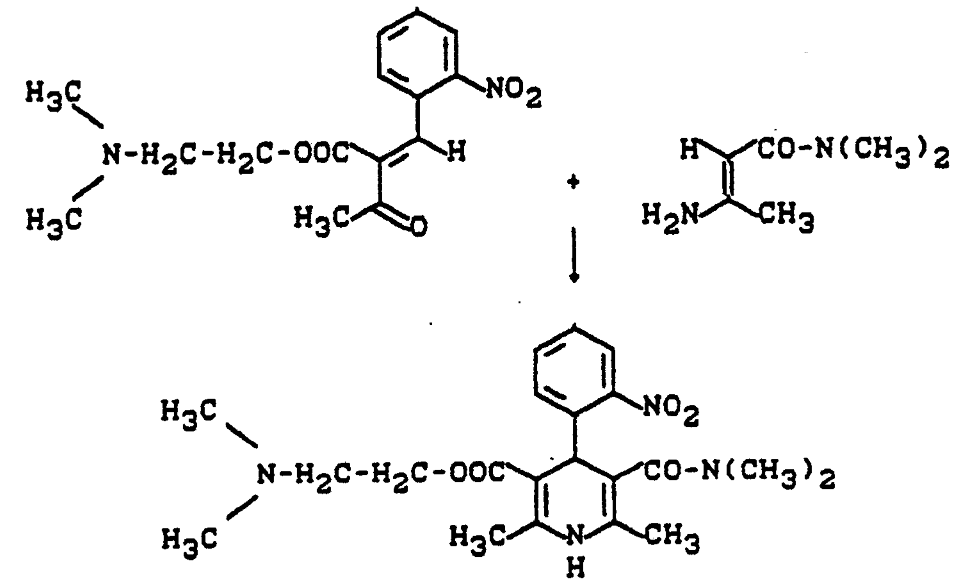

Variante B:

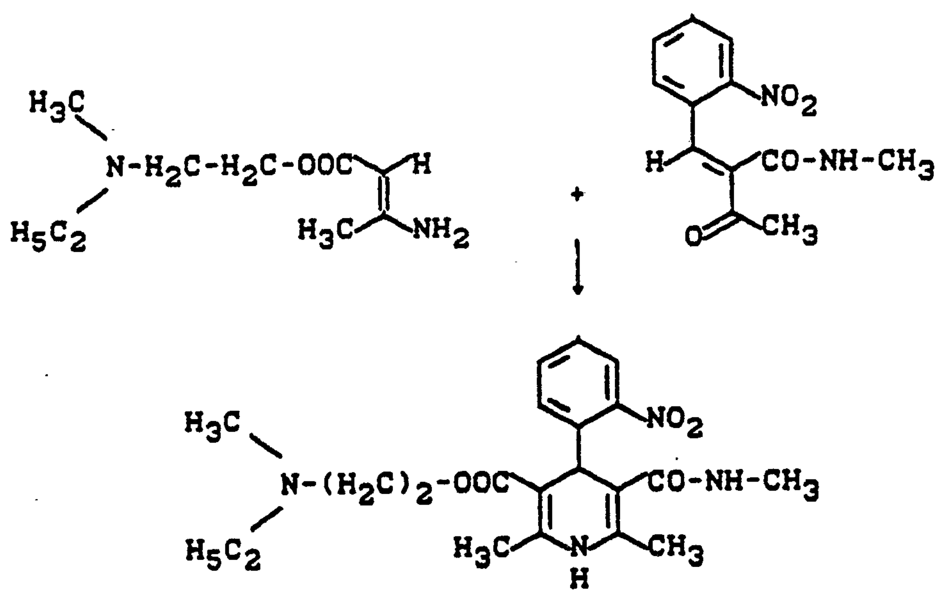

Variante D:

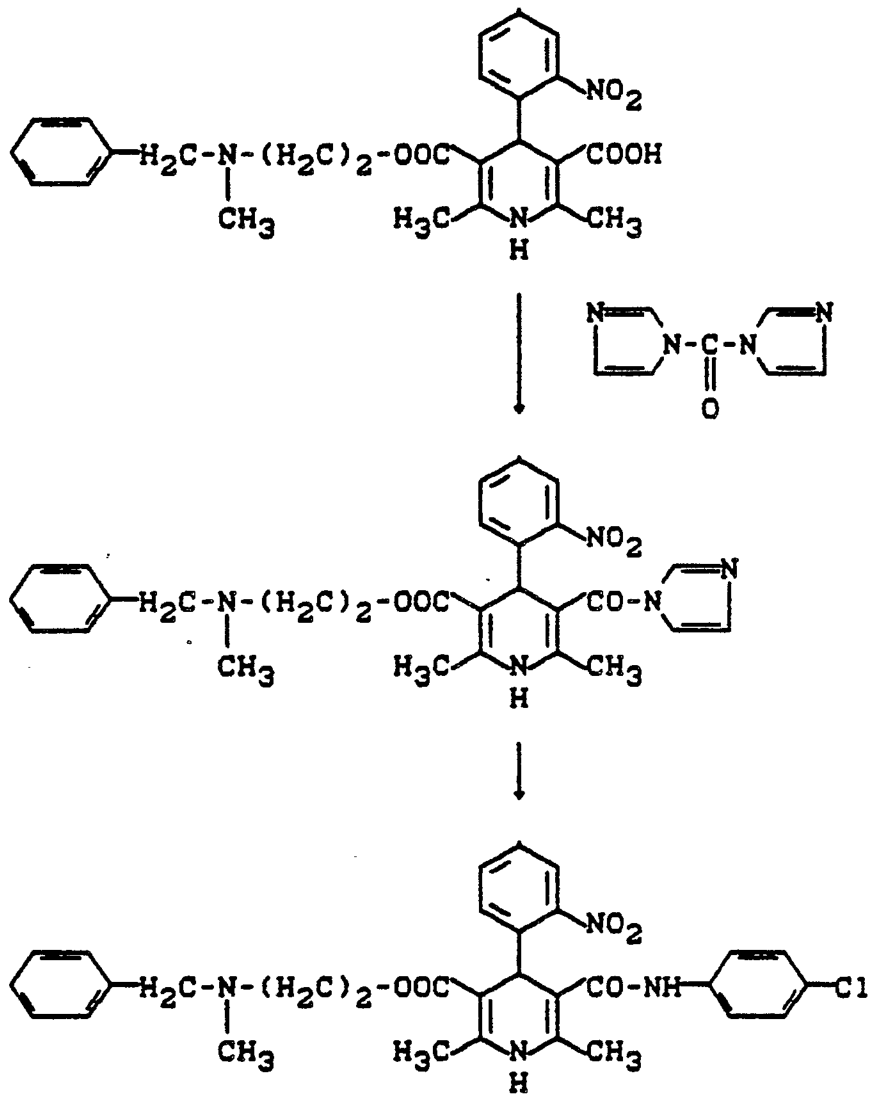

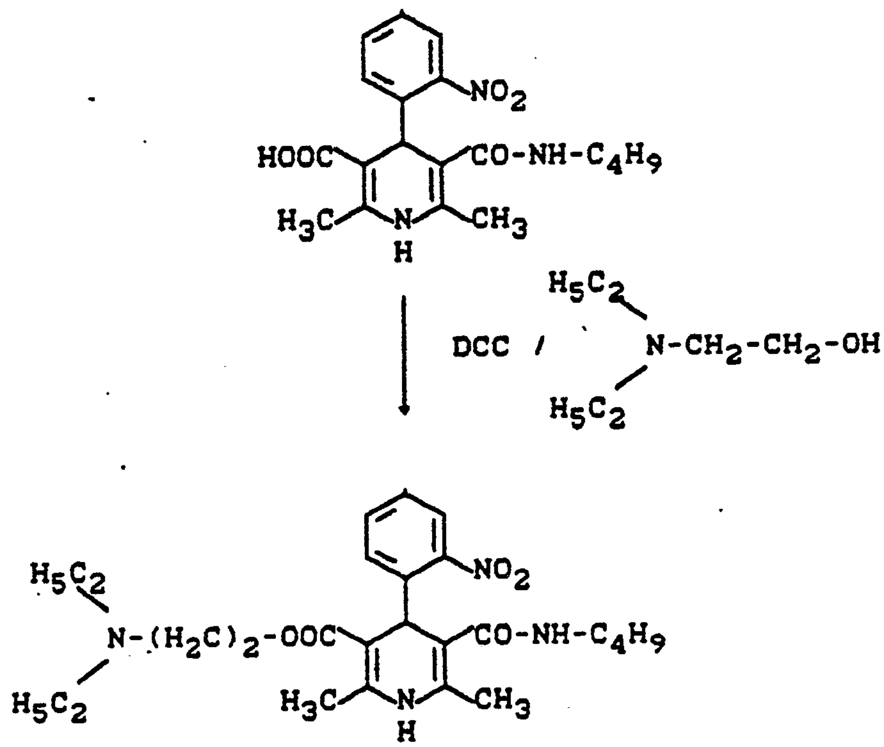


Verfahrensvarianten A - D

Als Lösemittel kommen Wasser oder alle inerten organischen Lösemittel in Frage, die sich unter den Reaktionsbedingungen nicht verändern. Hierzu gehören vorzugsweise Alkohole wie Methanol, Ethanol, Propanol, Isopropanol, Ether wie Diethylether, Dioxan, Tetrahydrofuran, Glykolmonomethylether oder Glykoldimethylether, oder Amide wie Dimethylformamid, Dimethylacetamid oder Hexamethylphosphorsäuretriamid, oder Eisessig, Dimethylsulfoxid, Acetonitril oder Pyridin.

Die Reaktionstemperaturen können in einem größeren Bereich variiert werden. Im allgemeinen arbeitet man zwischen +10° C und +150° C, vorzugsweise zwischen +20° C und +100° C, insbesondere bei der Siedetemperatur des jeweiligen Lösemittels.

Die Umsetzung kann bei Normaldruck, aber auch bei erhöhtem oder erniedrigtem Druck durchgeführt werden. Im allgemeinen arbeitet man bei Normaldruck.

Bei der Durchführung der erfindungsgemäßen Verfahrensvarianten A - D ist das Verhältnis der an der Reaktion beteiligten Stoffe beliebig. Im allgemeinen arbeitet man jedoch mit molaren Mengen der Reaktanden. Die Isolierung und Reinigung der erfindungsgemäßen Substanzen erfolgt vorzugsweise derart, daß man das Lösemittel im Vakuum abdestilliert und den gegebenenfalls erst nach Eiskühlung kristallin erhaltenen Rückstand aus einem geeigneten Lösemittel umkristallisiert. In einigen Fällen kann es erforderlich sein, die erfindungsgemäßen Verbindungen durch Chromatographie zu reinigen.

Die als Ausgangsstoffe eingesetzten Aldehyde der allgemeinen Formel (II) sind bekannt oder können nach bekannten Methoden hergestellt werden [DOS 21 65 260; 24 01 665; T.D. Harris, G.P. Roth, J. Org. Chem. 44, 2004 (1979); W.J. Dale, H.E. Hennis, J. Am. Chem. Soc. 78, 2543 (1956); Chem. Abstr. 59, 13929 (1963)].

Die als Ausgangsstoffe eingesetzten β-Ketocarbonsäureester der allgemeinen Formel (III) sind bekannt oder können nach bekannten Methoden hergestellt werden [D. Borrmann in Houben Weyl's "Methoden der organischen Chemie" Bd. VII/4, 230 (1968); Y. Oikawa, K. Sugano, O. Yonemitsu, J. Org. Chem. 43, 2087 (1978)].

Die als Ausgangsstoffe eingesetzten Enamine der allgemeinen Formeln (VI) und (VIII) sind bekannt oder können nach bekannten Methoden hergestellt werden [DOS 2 228 377; F.A. Glickman, A.C. Cope, J. Am. Chem. Soc. 67, 1017 (1945)].

Die Durchführung der erfindungsgemäßen Verfahrensvariante D lehnt sich an die literaturbekannte Methodik zur Überführung von Carbonsäuren in Carbonsäureamide an. Dabei wird die Carbonsäure

zunächst in eine aktivierte Form wie z.B. das Säurechlorid oder das Imidazolid überführt, die entweder als solche isoliert und in einem zweiten Reaktionsschritt umgesetzt werden, oder die in situ direkt zu den erfindungsgemäßen Verbindungen amidiert werden. Als aktivierende Reagenzien seien neben den anorganischen Halogeniden wie Thionylchlorid, Phosphortrichlorid oder Phosphorpentachlorid, oder dem Carbonyldiimidazol, Carbondiimide wie Cyclohexylcarbodiimid oder 1-Cyclohexyl-3-[2-(N-methyl-morpholino)ethyl]-carbodiimid-p-toluolsulfonat oder N-Hydroxyphthalimid oder N-Hydroxy-benztriazol in Gegenwart von Dicyclohexylcarbodiimid beispielhaft erwähnt. Naturgemäß lassen sich die Dihydropyridinmonocarbonsäuren auch in Form ihrer Salze einsetzen. [Die Methodik der Amidierung wird beispielsweise beschrieben: Fieser & Fieser, Reagents for Organic Synthesis, John Wiley & Sons Inc. (1967), Seite 231 -236; J.C. Shihan and G.P. Hess, J. Am. Chem. Soc. 77, 1067 (1955); U. Goodman, G.W. Kenner, Adv. in Protein Chem. 12, 488 (1957); W.A. Bonner, P.I. McNamee, J. Org. Chem. 26, 254 (1961); H.A. Staab, Angew. Chemie Int. Ed. 1, 351 (1962); Fieser & Fieser, Reagents for Organic Synthesis, John Wiley & Sons Inc. 1967, 116, 114; H.C. Beyerman, U.O. van der Brink, Re. Trav. 80, 1372 (1961); C.A. Buehler, D.E. Pearson, John Wiley & Sons, Volume I (1970), Seite 895 ff, Volume II, (1977)].

Als Lösemittel für Verfahrensvariante D kommen neben Wasser alle inerten organischen Lösemittel in Frage, die sich unter den Reaktionsbedingungen nicht verändern. Hierzu gehören vorzugsweise Ether wie Diethylether, Dioxan, Tetrahydrofuran, Glykolmonomethylether oder Glykoldimethylether, oder halogenierte Kohlenwasser stoffe wie Dichlormethan, Trichlormethan oder Tetrachlormethan, oder Amide wie Dimethylformamid, Dimethylacetamid oder Hexamethylphosphorsäuretriamid, oder Kohlenwasserstoffe wie Benzol, Toluol oder Xylol, oder Acetonitril, Nitromethan, Pyridin, Dimethylsulfoxid oder Essigester. Ebenso können Gemische der genannten Lösemittel verwendet werden. Isoliert man die aktivierten Zwischenstufen der Dihydropyridinmonocarbonsäuren, so können auch die Amine der Formel (XIa) alleine als Verdünnungsmittel verwendet werden.

Die Reaktionstemperaturen können in einem breiten Bereich variiert werden. Im allgemeinen arbeitet man in einem Bereich von -70 °C bis +140 °C, bevorzugt von -20 °C bis +100 °C.

Die Umsetzung kann bei Normaldruck, aber auch bei erhöhtem oder erniedrigtem Druck durchgeführt werden. Im allgemeinen arbeitet man bei Normaldruck.

Bei der Durchführung der erfindungsgemäßen Verfahrensvariante D ist das Verhältnis der an der Reaktion beteiligten Stoffe beliebig. Im allgemeinen arbeitet man jedoch mit molaren Mengen der Reaktanden. Es hat sich jedoch als günstig erwiesen, das Amin in einem 5- bis 10-fach molaren Überschuß einzusetzen. Besonders zweckmäßig wird das Amin in einem großen Überschuß direkt als Lösemittel eingesetzt.

Die Abspaltung der Aminoschutzgruppe erfolgt in an sich bekannter Weise, beispielsweise unter sauren Bedingungen, oder wenn Z für den Phthalimidrest steht, erfolgt die Abspaltung der Schutzgruppe üblicherweise mit Hydrazinhydrat in organischen Lösemitteln wie Ethern, z.B. Tetrahydrofuran oder Dioxan, oder Alkoholen z.B. Methanol, Ethanol oder Isopropanol.

Die als Ausgangsstoffe eingesetzten Amine der allgemeinen Formeln (XIa) und (XIb) sind bekannt oder können nach bekannten Methoden hergestellt werden [Houben Weyl's "Methoden der organischen Chemie" Bd XI/1; Paulsen, Angewandte Chemie 78, 501 - 566 (1966)].

Gegenstand der Erfindung sind auch die neuen Zwischenprodukte der allgemeinen Formeln (IX) und (IXa)

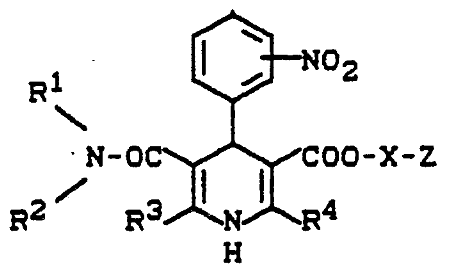

(IX)

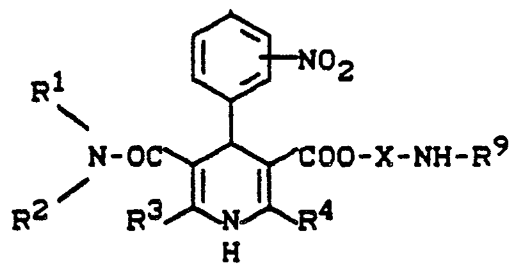

(IXa)

in welchen

$R^1$, $R^2$, $R^3$, $R^4$, X, Z und $R^9$ die oben angegebene Bedeutung haben.

Die Herstellung dieser neuen Zwischenprodukte der allgemeinen Formeln (IX) und (IXa) erfolgt indem man Benzylidenverbindungen der allgemeinen Formel (VII) mit Verbindungen der allgemeinen Formel (VIIIa) gegebenenfalls in Gegenwart von inerten organischen Lösungsmitteln cyclisiert und die erhaltene Verbindung der Formel (IX) gegebenenfalls durch Abspaltung der Schutzgruppe Z nach bekannten Methoden deblockiert zu Verbindungen (IXa).

Die neuen und die bekannten Verbindungen der Formel (I) und (Ia) zeigen ein nicht vorhersehbares, wertvolles pharmakologisches Wirkspektrum. Sie beeinflussen die Kontraktionskraft des Herzens, den Tonus der glatten Muskulatur sowie den Elektrolyt - und Flüssigkeitshaushalt.

Sie können deshalb in Arzneimitteln zur Behandlung des pathologisch veränderten Blutdrucks und der Herzinsuffizienz, sowie als Koronartherapeutika eingesetzt werden.

Darüber hinaus können sie zur Behandlung von Herzrhythmusstörungen, Niereninsuffizienz, Leberzirrhose, Aszites, Lungenödem, Hirnödem, Schwangerschaftsödem, Glaukom oder Diabetes mellitus eingesetzt werden.

Die Herzwirkung der erfindungsgemäßen Verbindungen wurde am isolierten, stimulierten Papillarmuskel des Meer schweinchenherzens gefunden. Dazu wurden die Versuchstiere (200 g schwere Meerschweinchen beiderlei Geschlechts) getötet, der Thorax geöffnet und das Herz entnommen. Für die Versuche wurden anschließend jeweils möglichst kleine Papillarmuskeln aus der rechten Herzkammer herauspräpariert und horizontal in einem Organbad fixiert. Dabei wurde das eine Ende des Muskels durch zwei Metallelektroden gehalten, die gleichzeitig zur Reizung des Präparates dienten, während das andere Ende des Muskels über einen Faden mit einem Kraftaufnehmer verbunden war. Der Papillarmuskel wurde mit einer Frequenz von 1 Hz überschwellig gereizt. Das Organbad mit einem Volumen von ca. 2 ml wurde kontinuierlich mit einer Krebs-Henseleit-Lösung (Konzentration in mM; NaCl 118; $NaHCO_3$ 25; KCl 10; $KH_2PO_4$ 1.2; $MgSO_4$ 1.2; $CaCl_2$ 1.8; Glukose 10, pH 7.4) mit einer Geschwindigkeit von 4 ml/min bei einer Temperatur von 32°C durchströmt. Die Kontraktionen des Papillarmuskels wurden isometrisch über den angeschlossenen Kraftaufnehmer gemessen und auf einem Schreiber registriert.

Die erfindungsgemäßen Stoffe wurden in der Krebs-Henseleit-Lösung in einer Konzentration von 10 µg/ml gegebenenfalls mit einem Lösungsvermittler (DMSO bis zu einer Konzentration von 0.5%) gelöst. Die erfindungsgemäßen Dihydropyridincarbonamide zeigten dabei bezogen auf die Kontrollwerte eine Hemmung der Kontraktionskraft des Papillarmuskels um mehr als 10%.

Zur Prüfung der renalen Wirkung wurden die Substanzen oral bei wachen männlichen Wistar-Ratten verabreicht. Nach Belastung mit physiologischer Kochsalzlösung wurde die Natriumausscheidung in Stoffwechselkäfigen gemessen.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nicht-toxische organische Lösungsmittel, wie Paraffine (z.B. Erdölfraktionen), pflanzliche Öle (z.B. Erdnuß/Sesamöl), Alkohole (z.b: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate, Arylsulfonate), Detergentien (z.B. Lignin-Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament , der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Herstellungsbeispiele

Verfahrensvariante D

Beispiel 1

1,4-Dihydro-2,6-dimethyl-4-(3-nitrophenyl)pyridin-3-carbonsäure-(2-N-benzyl-N-methyl-ethyl)-ester-5-carbonsäure-ethylamid-hydrochlorid

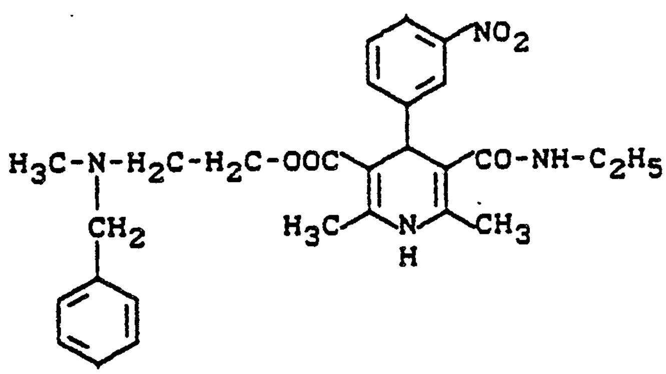

x HCl

4 g (7,5 mmol) 1,4-Dihydro-2,6-dimethyl-4-(3-nitrophenyl)-3-carbonsäure-(2-N-benzyl-N-methyl-ethylester)-5-carbonsäureimidazolid werden mit 40 ml 50%iger Ethylaminlösung über Nacht gerührt. Es wird eingeengt, in Essigester aufgenommen und 2 mal mit Wasser gewaschen, getrocknet und eingeengt. Der erhaltene Eindampfrückstand wird über eine Kieselgelsäule gereinigt. Die sauberen Fraktionen werden eingeengt und in das Hydrochlorid überführt. Man erhält 1,1 g farblose Kristalle vom Schmelzpunkt 150 °C unter Zersetzung.

Beispiel 2

Verfahrensvariante A

1,4-Dihydro-2,6-dimethyl-4-(2-nitrophenyl)-pyridin-3-carbonsäure-(β-N-morpholinoethyl)-ester-5-carbonsäurecyclopentylamid

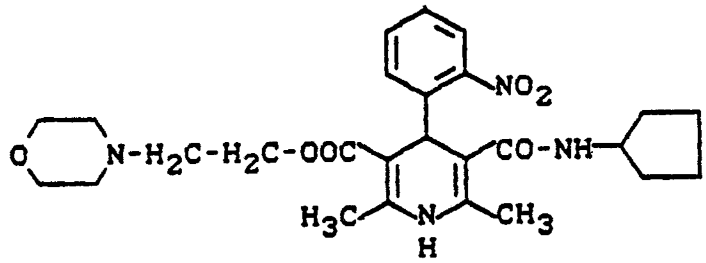

3,5 g (10 mmol) 2-Nitrobenzyliden-acetessigsäure-(β-N-morpholinoethyl)-ester werden in 25 ml Isopropanol mit 1,68 g (10 mmol) β-Aminocrotonsäurecyclopentylamid 4 Stunden unter Argon gekocht. Es wird abgekühlt, die ausgefallenen Kristalle werden abgesaugt und mit Isopropanol gewaschen. Man erhält 3,25 g orangefarbener Kristalle vom Schmelzpunkt 193 °C.

Analog der Vorschrift zu den Verfahrensvarianten A und D wurden die in der folgenden Tabelle aufgeführten Beispiele hergestellt:

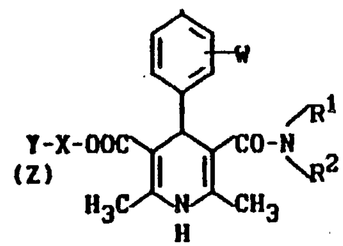

| Bsp.-Nr. | $R^1$ | $R^2$ | X | Y (Z) | W | Salz | Schmp. | $R_f$ |
|---|---|---|---|---|---|---|---|---|
| 3 | $CH_3$ | H | $(CH_2)_2$ | 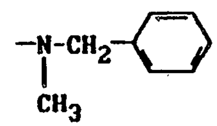 | $3-NO_2$ | - | Schaum | 0,38 a)<br>0,56 b) |
| 4 | 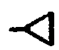 | H | $(CH_2)_2$ | 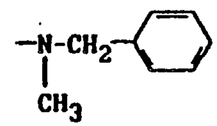 | $3-NO_2$ | HCl | 155° C | 0,42 a)<br>0,58 b) |
| 5 | $CH_3$ | H | $(CH_2)_2$ | 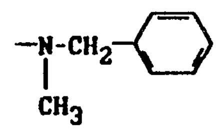 | $2-NO_2$ | - | 152-154° C | 0,43 a)<br>0,58 b) |
| 6 | 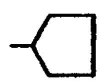 | H | $(CH_2)_2$ | 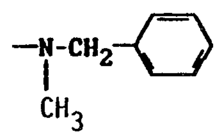 | $2-NO_2$ | - | 164-165° C | 0,55 a)<br>0,60 b) |
| 7 | 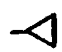 | H | $(CH_2)_2$ | 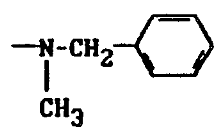 | $2-NO_2$ | - | 140-149° C | 0,46 a)<br>0,60 b) |

<u>**Fortsetzung Tabelle**</u>

| Bsp.-Nr. | $R^1$ | $R^2$ | X | Y | W | Salz | Schmp. | $R_f$ |
|---|---|---|---|---|---|---|---|---|
| 8 | $C_3H_7$ | H | $(CH_2)_2$ | 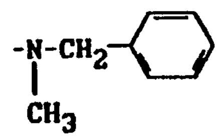 | 2-$NO_2$ | - | 163-165° C | 0,53 a)<br>0,01 b) |
| 9 | $CH_3$ | H | $(CH_2)_2$ | 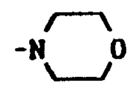 | 2-$NO_2$ | - | 154-157° C | 0,22 a)<br>0,48 b) |
| 10 | $CH_3$ | H | $(CH_2)_2$ | 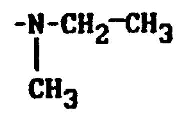 | 2-$NO_2$ | - | 128-130° C | 0,1 a)<br>0,16 b) |
| 11 | $C_3H_7$ | H | $(CH_2)_2$ | 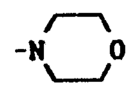 | 2-$NO_2$ | - | 139-141° C | 0,35 a)<br>0,56 b) |
| 12 | 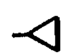 | H | $(CH_2)_2$ | 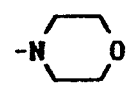 | 2-$NO_2$ | - | 129-131° C | 0,27 a)<br>0,54 b) |

**Fortsetzung Tabelle**

| Bsp.-Nr. | $R^1$ | $R^2$ | X | Y | W | Salz | Schmp. | $R_f$ |
|---|---|---|---|---|---|---|---|---|
| 13 | 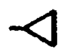 | H | $(CH_2)_2$ | 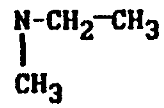 | 2-$NO_2$ | - | | 0,1 a) |
| 14 | 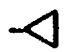 | H | $(CH_2)_2$ | 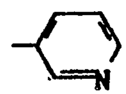 | 2-$NO_2$ | - | 196-198° C | 0,20 a)<br>0,553 b) |
| 15 | $CH_3$ | H | $(CH_2)_2$ | 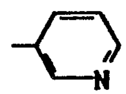 | 2-$NO_2$ | - | 192-194° C | 0,137 a)<br>0,523 b) |
| 16 | $C_2H_5$ | H | $(CH_2)_2$ | 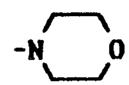 | 2-$NO_2$ | - | 147° C | 0,271 a)<br>0,53 b) |
| 17 | $C_2H_5$ | H | $(CH_2)_2$ | 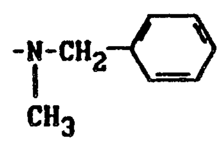 | 2-$NO_2$ | - | 147-149° C | |

**Fortsetzung Tabelle**

| Bsp.-Nr. | $R^1$ | $R^2$ | X | Y/Z | W | Salz | Schmp. | $R_f$ |
|---|---|---|---|---|---|---|---|---|
| 18 | $C_2H_5$ | H | $(CH_2)_2$ | 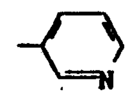 | 2-$NO_2$ | - | 160° C | |
| 19 | $C_2H_5$ | H | $(CH_2)_2$ | 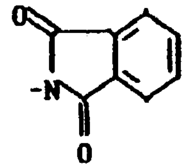 | 3-$NO_2$ | - | 108-110° C | 0,67 c) |
| 20 | 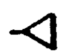 | H | $(CH_2)_2$ | 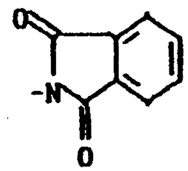 | 3-$NO_2$ | - | 123-125° C | 0,64 c) |
| 21 | $C_2H_5$ | H | $(CH_2)_2$ | 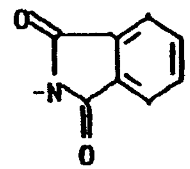 | 2-$NO_2$ | - | 120-122° C | 0,57 c) |
| 22 | $C_2H_5$ | H | $-CH(CH_3)-CH_2-$ | 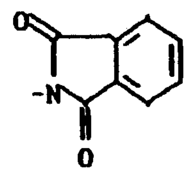 | 3-$NO_2$ | - | | 0,64 c) |

**Fortsetzung Tabelle**

| Bsp.-Nr. | $R^1$ | $R^2$ | X | Y/Z | W | Salz | Schmp. | $R_f$ |
|---|---|---|---|---|---|---|---|---|
| 23 | 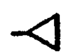 | H | $CH_3$<br>$-CH-CH_2-$ | 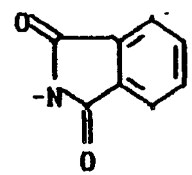 | $3-NO_2$ | - | 127-131°C | 0,60 c) |
| 24 | $C_2H_5$ | H | $CH_3$<br>$-CH-CH_2-$ | 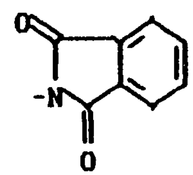 | $2-NO_2$ | - | | |

Beispiel 25

1,4-Dihydro-2,6-dimethyl-4-(3-nitrophenyl)-pyridin-3-carbonsäure-(2-aminomethyl)-ester-5-carbonsäure-

cyclopropylamid

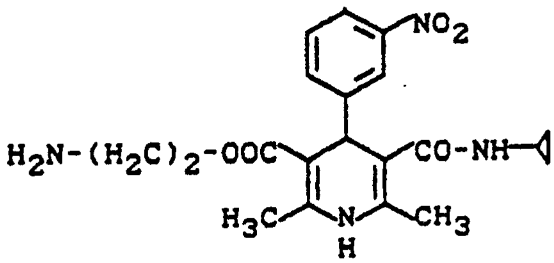

Eine Lösung von 9.7 g (18,7 mmol) 1,4-Dihydro-2,6-dimethyl-4-(3-nitrophenyl)-pyridin-3-carbonsäure-(2-phthalimidoethyl)-ester-5-carbonsäure-cyclopropylamid (Beispiel 20) und 93,5 mmol Hydrazinhydrat in 200 ml Ethanol wurde 1 h unter Rückfluß gekocht. Die Mischung wurde abgekühlt und der Niederschlag abfiltriert. Anschließend wurde der Rückstand mit Methylenchlorid gewaschen und die organische Phase im Vakuum eingeengt. Nach Zugabe von Methylenchlorid wurde einmal mit einer 2 N Lösung von Kaliumhydroxid und 3 mal mit Wasser gewaschen. Die organische Phase wurde mit Natriumsulfat getrocknet und im Vakuum eingeengt. Das Produkt kristallisiert beim Verreiben mit Ether.
Ausbeute: 5,95 g (82% der Theorie)
Schmelzpunkt: 55 - 57 °C
$R_f$: 0,17 [d)]

Analog der Vorschrift von Beispiel 25 wurden die in Tabelle 2 aufgeführten Verbindungen hergestellt:

**Tabelle 2**

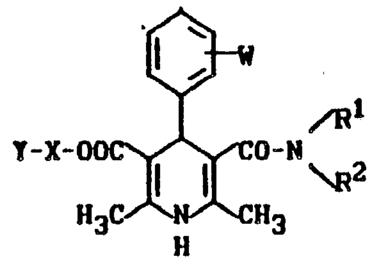

| Bsp.-Nr. | $R^1$ | $R^2$ | X | Y | W | Salz | Schmp. | $R_f$ |
|---|---|---|---|---|---|---|---|---|
| 26 | $C_2H_5$ | H | $-(CH_2)_2$ | $-NH_2$ | $3-NO_2$ | - | 144-146° C | 0,18 d) |
| 27 | $C_2H_5$ | H | $CH_3$<br>$-CH-CH_2-$ | $-NH_2$ | $3-NO_2$ | - | 169-173° C<br>Beide diastereomeren Formen | 0,52 d) |
| 28 | 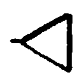 | H | $CH_3$<br>$-CH-CH_2-$ | $-NH_2$ | $3-NO_2$ | - | 185-188° C<br>Beide diastereomeren Formen | |

Folgende Lösemittel wurden unter a) - d) aufgeführt:

a) Toluol/Aceton (1:1)
b) Toluol/Ethanol (3:1)
c) Methylenchlorid/Methanol (10:1)
d) Methylenchlorid/Methanol (5:1)

## Ansprüche

1. 4-Nitrophenyl-dihydropyridinamide der allgemeinen Formel (I)

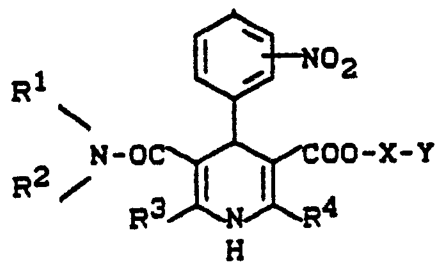

(I)

in welcher
$R^1$ und $R^2$ gleich oder verschieden sind und
- Wasserstoff bedeuten, oder
- für Cycloalkyl mit 3 bis 8 Kohlenstoffatomen stehen, oder
- für geradkettiges oder verzweigtes Alkyl oder Alkenyl, mit jeweils bis zu 12 Kohlenstoffatomen stehen, die gegebenenfalls substituiert sind durch Halogen, Hydroxy, Alkoxy mit bis zu 8 Kohlenstoffatomen, Alkylthio mit bis zu 8 Kohlenstoffatomen, Alkylcarbonyl mit bis zu 8 Kohlenstoffatomen, Carboxy oder Alkoxycarbonyl mit bis zu 8 Kohlenstoffatomen oder durch Phenyl, das seinerseits durch Halogen, Nitro, Cyano, Trifluormethyl, Trifluormethoxy, Alkyl mit bis zu 4 Kohlenstoffatomen oder Alkoxy mit bis zu 4 Kohlenstoffatomen oder durch eine Gruppe $-NR^5R^6$ substituiert sein kann,
worin
$R^5$ und $R^6$ gleich oder verschieden sind und Wasserstoff, Alkyl mit bis zu 8 Kohlenstoffatomen, Aryl oder Aralkyl bedeuten,
- für Phenyl stehen, das bis zu 3-fach gleich oder verschieden durch Nitro, Cyano, Halogen, Alkyl mit bis zu 6 Kohlenstoffatomen, Alkoxy mit bis zu 6 Kohlenstoffatomen, Alkylthio mit bis zu 6 Kohlenstoffatomen, Trifluormethyl, Trifluormethoxy, Difluormethoxy, Trifluormethylthio, Amino, Alkylamino mit bis zu 8 Kohlenstoffatomen je Alkylgruppe, Dialkylamino mit bis zu 8 Kohlenstoffatomen je Alkylgruppe, Acetylamino oder Benzoylamino substituiert sein kann, oder
$R^1$ und $R^2$ gemeinsam einen 5- bis 7-gliedrigen gesättigten oder ungesättigten, heterocyclischen Ring bilden, der als zusätzliches Heteroatom ein Sauerstoffatom, ein Schwefelatom, ein Stickstoffatom, oder einen Rest $N-R^7$ enthalten kann,
worin
$R^7$ - Wasserstoff, $C_1$-$C_6$-Alkyl, Aryl oder Aralkyl bedeuten kann
und
$R^3$ und $R^4$ gleich oder verschieden sind und
- für geradkettiges, verzweigtes oder cyclisches Alkyl mit bis zu 8 Kohlenstoffatomen stehen, das gegebenenfalls durch Hydroxy, Cyano, Phenyl oder Halogen substituiert ist
und
X - für einen geradkettigen, verzweigten oder cyclischen, gesättigten oder ungesättigten Kohlenwasserstoffrest mit bis zu 12 Kohlenstoffatomen steht, der gegebenenfalls durch ein Sauerstoff-, Schwefel-oder Stickstoffatom, oder eine Gruppe $N-R^8$ unterbrochen ist,
worin
$R^8$ - Wasserstoff bedeutet, oder
- für Alkyl mit bis zu 6 Kohlenstoffatomen steht, das gegebenenfalls substituiert sein kann durch Halogen, Hydroxy, Acetoxy, Carboxy, $C_1$-$C_8$-Alkoxycarbonyl oder Phenyl, das seinerseits substituiert sein kann durch Halogen, $C_1$-$C_6$-Alkyl, $C_1$-$C_6$-Alkoxy, Halogenmethyl, Halogenmethoxy oder Cyano,
und
Y - Pyridyl bedeutet, oder
- für eine Gruppe der Formel $-NR^9R^{10}$ steht,
worin
$R^9$ und $R^{10}$ gleich oder verschieden sind und
- Wasserstoff bedeuten, oder
- für geradkettiges oder verzweigtes Alkyl, Alkenyl oder Alkinyl mit jeweils bis zu 12 Kohlenstoffatomen stehen, das substituiert sein kann durch Halogen, Hydroxy, Alkoxy mit bis zu 8 Kohlenstoffatomen, Cyano, Trifluormethyl, Alkylthio mit bis zu 8 Kohlenstoffatomen, Alkylcarbonyl mit bis zu 8 Kohlenstoffatomen, Alkoxycarbonyl mit bis zu 8 Kohlenstoffatomen oder durch Phenyl, welches seinerseits durch Nitro, Phenyl, Cyano, Trifluormethyl, Trifluormethoxy, Alkyl mit bis zu 4 Kohlenstoffatomen, Halogen oder Alkoxy mit bis zu 4 Kohlenstoffatomen substituiert sein kann, oder

- für Cycloalkyl mit 3 bis 8 Kohlenstoffatomen stehen, oder
- für Aryl mit 6 bis 10 Kohlenstoffatomen stehen, das bis zu 3-fach gleich oder verschieden durch Nitro, Cyano, Halogen, Alkyl mit bis zu 6 Kohlenstoffatomen, Alkoxy mit bis zu 6 Kohlenstoffatomen, Alkylthio mit bis zu 6 Kohlenstoffatomen, Carbamoyl, Dialkylcarbamoyl mit jeweils bis zu 6 Kohlenstoffatomen je Alkylgruppe, Trifluormethyl, Trifluormethoxy, Difluormethoxy, Trifluormethylthio, Amino, Alkylamino mit bis zu 8 Kohlenstoffatomen, Dialkylamino mit jeweils bis zu 8 Kohlenstoffatomen je Alkylgruppe substituiert sein kann, oder

$R^9$ und $R^{10}$ zusammen einen 5- bis 7-gliedrigen, gesättigten oder ungesättigten, heterocyclischen Ring bilden, der ein Sauerstoffatom, ein Schewefelatom oder ein zusätzliches Stickstoffatom enthalten kann, das gegebenenfalls durch einen Rest $R^{11}$ substituiert ist,

worin

$R^{11}$ - für Wasserstoff steht, oder
- für eine geradkettige oder verzweigte, gesättigte oder ungesättigte Alkylengruppe mit bis zu 10 Kohlenstoffatomen steht, die gegebenenfalls durch Phenyl substituiert ist, welches seinerseits durch Halogen, $C_1$-$C_4$-Alkyl, $C_1$-$C_4$-Alkoxy, Nitro oder Halogenalkyl mit bis zu 4 Kohlenstoffatomen substituiert sein kann, oder
- für Phenyl steht, das gegebenenfalls durch Halogen, Cyano, Nitro, Alkyl mit bis zu 4 Kohlenstoffatomen, Alkoxy mit bis zu 4 Kohlenstoffatomen oder Halogenalkyl mit bis zu 4 Kohlenstoffatomen substituiert ist,

und deren physiologisch unbedenklichen Salze zur Verwendung bei der Bekämpfung der Hypertonie und der Herzinsuffizienz.

2. Verbindungen der allgemeinen Formel (I), gemäß Anspruch 1, in welcher

$R^1$ und $R^2$ gleich oder verschieden sind und
- Wasserstoff bedeuten, oder
- für Cycloalkyl mit 3 bis 8 Kohlenstoffatomen stehen, oder
- für geradkettiges oder verzweigtes Alkyl oder Alkenyl, mit jeweils bis zu 10 Kohlenstoffatomen stehen, die gegebenenfalls substituiert sind durch Fluor, Chlor, Hydroxy, $C_1$-$C_6$-Alkoxy, $C_1$-$C_6$-Alkylthio, $C_1$-$C_6$-Alkylcarbonyl, $C_1$-$C_6$-Alkoxycarbonyl, Carboxy oder durch Phenyl, welches seinerseits durch Halogen, Nitro, Cyano, Trifluormethyl, Trifluormethoxy, Methyl, Methoxy oder durch eine Gruppe der Formel $-NR^5R^6$ substituiert sein kann,

worin

$R^5$ und $R^6$ gleich oder verschieden sind und
- für Wasserstoff, $C_1$-$C_6$-Alkyl, Benzyl, Phenethyl oder Phenyl stehen,
- für Phenyl stehen, das gegebenenfalls bis zu 2-fach gleich oder verschieden substituiert ist durch Fluor, Chlor, $C_1$-$C_4$-Alkyl, $C_1$-$C_4$-Alkoxy, $C_1$-$C_4$-Alkylthio, Trifluormethyl, Trifluormethoxy, Difluormethoxy, oder

$R^1$ und $R^2$ gemeinsam für Pyridyl, Pyrrolidyl, Piperidyl, Morpholinyl, oder
- für gegebenenfalls durch $C_1$-$C_4$-Alkyl, Benzyl oder Phenethyl substituiertes Piperazinyl stehen,

$R^3$ und $R^4$ gleich oder verschieden sind und
- für geradkettiges oder verzweigtes Alkyl mit bis zu 6 Kohlenstoffatomen stehen, das gegebenenfalls durch Fluor, Chlor, Phenyl oder Hydroxy substituiert ist,

X - für einen geradkettigen, verzweigten oder cyclischen, gesättigten oder ungesättigten Kohlenwasserstoffrest mit bis zu 10 Kohlenstoffatomen steht, der gegebenenfalls durch ein Sauerstoff-, ein Schwefel- oder ein Stickstoffatom, oder durch einen Rest $N-R^8$ unterbrochen ist,

worin

$R^8$ - Wasserstoff bedeutet, oder
- für $C_1$-$C_4$-Alkyl steht, das gegebenenfalls substituiert ist durch Fluor, Chlor, Hydroxy, Carboxy oder durch Phenyl, welches seinerseits durch Fluor, Chlor, $C_1$-$C_4$-Alkyl, $C_1$-$C_4$-Alkoxy, Trifluormethyl oder Trifluormethoxy substituiert sein kann,

und

Y - Pyridyl bedeutet, oder
- für eine Gruppe der Formel $-NR^9R^{10}$ steht,

worin

$R^9$ und $R^{10}$ gleich oder verschieden sind und
- Wasserstoff bedeuten, oder
- für geradkettiges oder verzweigtes Alkyl oder Alkenyl mit jeweils bis zu 8 Kohlenstoffatomen stehen, das substituiert sein kann durch Fluor, Chlor, Hydroxy, $C_1$-$C_4$-Alkoxy, $C_1$-$C_4$-Alkylthio, $C_1$-$C_4$-Alkylcarbonyl, Carboxy oder $C_1$-$C_6$-Alkoxycarbonyl, oder durch Phenyl, welches seinerseits durch Trifluormethyl, $C_1$-$C_2$-Alkyl, Fluor, Chlor oder $C_1$-$C_2$-Alkoxy substituiert sein kann, oder
- für Cycloalkyl mit 3 bis 8 Kohlenstoffatomen stehen, oder
- für Phenyl stehen, das bis zu 2-fach gleich oder verschieden durch Nitro, Cyano, Fluor, Chlor, Brom, $C_1$-

$C_4$-Alkyl, $C_1$-$C_4$-Alkoxy, $C_1$-$C_4$-Alkylthio, Trifluormethyl, Trifluormethoxy, Difluormethoxy, Trifluormethylthio, Amino, Alkylamino mit bis zu 4 Kohlenstoffatomen substituiert sein kann, oder
$R^9$ und $R^{10}$ zusammen einen 5- bis 7-gliedrigen gesättigten oder ungesättigten, heterocyclischen Ring bilden, der ein Sauerstoff- oder Schwefelatom oder ein zusätzliches Stickstoffatom enthalten kann, welches gegebenenfalls durch einen Rest $R^{11}$ substituiert ist,
worin
$R^{11}$ - für Wasserstoff steht, oder
- für eine geradkettige oder verzweigte, gesättigte oder ungesättigte Alkylgruppe mit bis zu 4 Kohlenstoffatomen steht, die gegebenenfalls durch Phenyl substituiert ist, welches seinerseits durch Fluor, Chlor, $C_1$-$C_2$-Alkyl, $C_1$-$C_2$-Alkoxy oder $C_1$-$C_2$-Halogenalkyl substituiert sein kann, oder
- für Phenyl steht, das gegebenenfalls durch Fluor, Chlor, Cyano, $C_1$-$C_2$-Alkyl, $C_1$-$C_2$-Alkoxy oder $C_1$-$C_2$-Halogenalkyl substituiert ist
und deren physiologisch unbedenklichen Salze zur Verwendung bei der Bekämpfung der Hypertonie und der Herzinsuffizienz.

3. Verbindungen der allgemeinen Formel (I) gemäß Anspruch 1
in welcher
$R^1$ und $R^2$ gleich oder verschieden sind und
- für Wasserstoff, Cyclopropyl, Cyclopentyl oder Cyclohexyl stehen, oder
- für geradkettiges oder verzweigtes Alkyl oder Alkenyl mit bis zu 8 Kohlenstoffatomen stehen, das gegebenenfalls substituiert ist durch Fluor, Chlor, Hydroxy, $C_1$-$C_4$-Alkoxy, $C_1$-$C_4$-Alkylthio, Carboxy, $C_1$-$C_4$-Alkoxycarbonyl oder durch Phenyl, das seinerseits durch Chlor, Fluor, Methyl oder Methoxy substituiert sein kann, oder
- für Phenyl stehen, das substituiert sein kann durch Fluor, Chlor, Methyl, Ethyl, Methoxy, Trifluormethyl, Trifluormethoxy,
$R^3$ und $R^4$ gleich oder verschieden sind und
- für Methyl stehen
und
X - für einen geradkettigen, verzweigten oder cyclischen, gesättigten oder ungesättigten Kohlenwasserstoffrest mit bis zu 8 Kohlenstffatomen steht, der gegebenenfalls durch ein Sauerstoff-, Schwefel-oder Stickstoffatom, oder durch einen Rest -N-$R^8$ unterbrochen ist,
worin
$R^8$ - Wasserstoff bedeutet, oder
- für Methyl, Ethyl, Benzyl, Phenethyl oder Phenyl steht, das gegebenenfalls Fluor, Chlor, Methyl, Methoxy, Trifluormethyl oder Trifluormethoxy substituiert ist,
Y - für Pyridyl steht, oder
- für eine Gruppe der Formel -$NR^9R^{10}$ steht,
worin
$R^9$ und $R^{10}$ gleich oder verschieden sind und
- Wasserstoff bedeuten, oder
- für geradkettiges oder verzweigtes Alkyl oder Alkenyl mit bis zu 4 Kohlenstoffatomen stehen, das substituiert sein kann durch Fluor, Chlor, Hydroxy, $C_1$-$C_4$-Alkoxycarbonyl oder durch Phenyl, das seinerseits durch Fluor, Chlor, Trifluormethyl, Methyl oder Methoxy substituiert sein kann, oder
- für Cyclopropyl, Cyclopentyl oder Cyclohexyl stehen, oder
- für Phenyl stehen, das bis zu 2-fach gleich oder verschieden durch Fluor, Chlor, Methyl, Ethyl, Methoxy, Ethoxy, Methylthio, Trifluormethyl, Trifluormethoxy, Difluormethoxy, Amino, $C_1$-$C_2$-Alkylamino oder $C_1$-$C_2$-Dialkylamino substituiert sein kann, oder
$R^9$ und $R^{10}$ gemeinsam einen 5- bis 7-gliedrigen gesättigten Ring bilden, der ein Sauerstoff-, Schwefel- oder ein zusätzliches Stickstoffatom enthalten kann, das gegebenenfalls durch einen Rest $R^{11}$ substituiert ist,
worin
$R^{11}$ - für Wasserstoff, $C_1$-$C_4$-Alkyl, Benzyl oder Phenethyl steht, oder
- für Phenyl steht, das durch Fluor, Chlor, Methyl oder Methoxy substituiert sein kann
und deren physiologisch unbedenklichen Salze zur Verwendung bei der Bekämpfung der Hypertonie und der Herzinsuffizienz.

4. Verwendung von Verbindungen der allgemeinen Formel (I) gemäß Anspruch 1 bei der Herstellung von Arzneimitteln zur Bekämpfung von Hypertonie, Herzrythmusstörungen, Niereninsuffizienz, Leberzirrhose, Aszites, Ödemen, Glaukom und Diabetes.

5. Arzneimittel enthaltend mindestens eine Verbindung der allgemeinen Formel (I) gemäß Anspruch 1.

6. Verfahren zur Herstellung von Arzneimitteln, dadurch gekennzeichnet, daß man mindestens eine Verbindung der allgemeinen Formel (I) gemäß Anspruch 1 gegebenenfalls unter Verwendung üblichen Hilfs- und Trägerstoffen in eine geeignete Applikationsform überführt.

7. 4-Nitrophenyl-dihydropyridinamide der allgemeinen Formel (Ia)

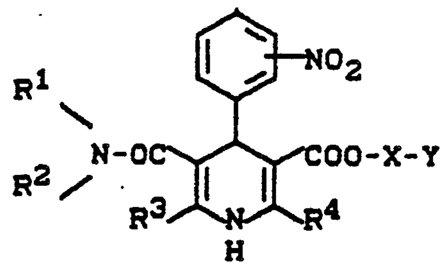

(Ia)

in welcher
$R^1$ und $R^2$ gleich oder verschieden sind und
- Wasserstoff bedeuten, oder
- für Cycloalkyl mit 3 bis 8 Kohlenstoffatomen stehen, oder
- für geradkettiges oder verzweigtes Alkyl oder Alkenyl, mit jeweils bis zu 12 Kohlenstoffatomen stehen, die gegebenenfalls substituiert sind durch Halogen, Hydroxy, Alkoxy mit bis zu 8 Kohlenstoffatomen, Alkylthio mit bis zu 8 Kohlenstoffatomen, Alkylcarbonyl mit bis zu 8 Kohlenstoffatomen, Carboxy oder Alkoxycarbonyl mit bis zu 8 Kohlenstoffatomen oder durch Phenyl, das seinserseits durch Halogen, Nitro, Cyano, Trifluormethyl, Trifluormethoxy, Alkyl mit bis zu 4 Kohlenstoffatomen oder Alkoxy mit bis zu 4 Kohlenstoffatomen oder durch eine Gruppe $-NR^5R^6$ substituiert sein kann,
worin
$R^5$ und $R^6$ gleich oder verschieden sind und Wasserstoff, Alkyl mit bis zu 8 Kohlenstoffatomen bedeuten,
- für Phenyl stehen, das bis zu 3-fach gleich oder verschieden durch Nitro, Cyano, Halogen, Alkyl mit bis zu 6 Kohlenstoffatomen, Alkoxy mit bis zu 6 Kohlenstoffatomen, Alkylthio mit bis zu 6 Kohlenstoffatomen, Trifluormethyl, Trifluormethoxy, Difluormethoxy, Trifluormethylthio, Amino, Alkylamino mit bis zu 8 Kohlenstoffatomen, Dialkylamino mit bis zu 8 Kohlenstoffatomen je Alkylgruppe, Acetylamino oder Benzoylamino substituiert sein kann, oder
$R^1$ und $R^2$ gemeinsam einen 5- bis 7-gliedrigen gesättigten oder ungesättigten, heterocyclischen Ring bilden, der als zusätzliches Heteroatom ein Sauerstoffatom, ein Schwefelatom, ein Stickstoffatom, oder einen Rest $N-R^7$ enthalten kann,
worin
$R^7$ - Wasserstoff, $C_1$-$C_6$-Alkyl, Aryl oder Aralkyl bedeuten kann
und
$R^3$ und $R^4$ gleich oder verschieden sind und
- für geradkettiges, verzweigtes oder cyclisches Alkyl mit bis zu 8 Kohlenstoffatomen stehen, das gegebenenfalls durch Hydroxy, Cyano, Phenyl oder Halogen substituiert ist
und
X - für einen geradkettigen, verzweigten oder cyclischen, gesättigten oder ungesättigten Kohlenwasserstoffrest mit bis zu 12 Kohlenstoff atomen steht, der gegebenenfalls durch ein Sauerstoff-, Schwefel- oder Stickstoffatom, oder eine Gruppe $N-R^8$ unterbrochen ist,
worin
$R^8$ - Wasserstoff bedeutet, oder
- für Alkyl mit bis zu 6 Kohlenstoffatomen steht, das gegebenenfalls substituiert sein kann durch Halogen, Hydroxy, Acetoxy, Carboxy, $C_1$-$C_8$-Alkoxycarbonyl oder Phenyl, das seinerseits substituiert sein kann durch Halogen, $C_1$-$C_6$-Alkyl, $C_1$-$C_6$-Alkoxy, Halogenmethyl, Halogenmethoxy oder Cyano,
und
Y- Pyridyl bedeutet, oder
- für eine Gruppe der Formel $-NR^9R^{10}$ steht,
worin
$R^9$ und $R^{10}$ gleich oder verschieden sind und
- für Wasserstoff oder geradkettiges oder verzweigtes Alykl, Alkenyl oder Alkinyl mit jeweils bis zu 12 Kohlenstoffatomen stehen, das substituiert sein kann durch Halogen, Hydroxy, Alkoxy mit bis zu 8 Kohlenstoffatomen, Cyano, Trifluormethyl, Alkylthio mit bis zu 8 Kohlenstoffatomen, Alkylcarbonyl mit bis zu

8 Kohlenstoffatomen, Alkoxycarbonyl mit bis zu 8 Kohlenstoffatomen oder durch Phenyl, welches seinerseits durch Nitro, Phenyl, Cyano, Trifluormethyl, Trifluormethoxy, Alkyl mit bis zu 4 Kohlenstoffatomen, Halogen, oder Alkoxy mit bis zu 4 Kohlenstoffatomen substituiert sein kann, oder
- für Cycloalkyl mit 3 bis 8 Kohlenstoffatomen stehen, oder
- für Aryl mit 6 bis 10 Kohlenstoffatomen stehen, das bis zu 3-fach gleich oder verschieden durch Nitro, Cyano, Halogen, Alkyl mit bis zu 6 Kohlenstoffatomen, Alkoxy mit bis zu 6 Kohlenstoffatomen, Alkylthio mit bis zu 6 Kohlenstoffatomen, Carbamoyl, Dialkylcarbamoyl mit jeweils bis zu 6 Kohlenstoffatomen je Alkylgruppe, Trifluormethyl, Trifluormethoxy, Difluormethoxy, Trifluormethylthio, Amino, Alkylamino mit bis zu 8 Kohlenstoffatomen, Dialkylamino mit jeweils bis zu 8 Kohlenstoffatomen je Alkylgruppe substituiert sein kann, oder
$R^9$ und $R^{10}$ zusammen einen 5- bis 7-gliedrigen, gesättigten oder ungesättigten, heterocyclischen Ring bilden, der ein Sauerstoffatom, ein Schwefelatom oder ein zusätzliches Stickstoffatom enthalten kann, das gegebenenfalls durch einen Rest $R^{11}$ substituiert ist,
worin
$R^{11}$ - für Wasserstoff steht, oder
- für eine geradkettige oder verzweigte, gesättigte oder ungesättigte Alkylengruppe mit bis zu 10 Kohlenstoffatomen steht, die gegebenenfalls durch Phenyl substituiert ist, welches seinerseits durch Halogen, $C_1$-$C_4$-Alkyl, $C_1$-$C_4$-Alkoxy, Nitro oder Halogenalkyl mit bis zu 4 Kohlenstoffatomen substituiert sein kann, oder
- für Phenyl steht, das gegebenenfalls durch Halogen, Cyano, Nitro, Alkyl mit bis zu 4 Kohlenstoffatomen, Alkoxy mit bis zu 4 Kohlenstoffatomen oder Halogenalkyl mit bis zu 4 Kohlenstoffatomen substituiert ist,
und deren physiologisch unbedenklichen Salze.

8. Verbindungen der allgemeinen Formel (Ia) gemäß Anspruch 7
in welcher
$R^1$ und $R^2$ gleich oder verschieden sind und
- Wasserstoff bedeuten, oder
- für Cycloalkyl mit 3 bis 8 Kohlenstoffatomen stehen, oder
- für geradkettiges oder verzweigtes Alkyl oder Alkenyl, mit jeweils bis zu 10 Kohlenstoffatomen stehen, die gegebenenfalls substituiert sind durch Fluor, Chlor, Hydroxy, $C_1$-$C_6$-Alkoxy, $C_1$-$C_6$-Alkylthio, $C_1$-$C_6$-Alkylcarbonyl, $C_1$-$C_6$-Alkoxycarbonyl, Carboxy oder durch Phenyl, welches seinerseits durch Halogen, Nitro, Cyano, Trifluormethyl, Trifluormethoxy, Methyl, Methoxy oder durch eine Gruppe der Formel $-NR^5R^6$ substituiert sein kann,
worin
$R^5$ und $R^6$ gleich oder verschieden sind und
- für Wasserstoff, $C_1$-$C_6$-Alkyl, Benzyl, Phenethyl oder Phenyl stehen,
- für Phenyl stehen, das gegebenenfalls bis zu 2-fach gleich oder verschieden substituiert ist durch Fluor, Chlor, $C_1$-$C_4$-Alkyl, $C_1$-$C_4$-Alkoxy, $C_1$-$C_4$-Alkylthio, Trifluormethyl, Trifluormethoxy, Difluormethoxy, oder
$R^1$ und $R^2$ gemeinsam für Pyridyl, Pyrrolidyl, Piperidyl, Morpholinyl, oder
- für gegebenenfalls durch $C_1$-$C_4$-Alkyl, Benzyl oder Phenethyl substituiertes Piperazinyl stehen,
$R^3$ und $R^4$ gleich oder verschieden sind und
- für geradkettiges oder verzweigtes Alkyl mit bis zu 6 Kohlenstoffatomen stehen, das gegebenenfalls durch Fluor, Chlor, Phenyl oder Hydroxy substituiert ist,
X - für einen geradkettigen, verzweigten oder cyclischen, gesättigten oder ungesättigten Kohlenwasserstoffrest mit bis zu 10 Kohlenstoffatomen steht, der gegebenenfalls durch ein Sauerstoff-, ein Schwefel- oder ein Stickstoffatom, oder durch einen Rest $N-R^8$ unterbrochen ist,
worin
$R^8$ - Wasserstoff bedeutet, oder
- für $C_1$-$C_4$-Alkyl steht, das gegebenenfalls substituiert ist durch Fluor, Chlor, Hydroxy, Carboxy oder durch Phenyl, welches seinerseits durch Fluor, Chlor, $C_1$-$C_4$-Alkyl, $C_1$-$C_4$-Alkoxy, Trifluormethyl oder Trifluormethoxy substituiert sein kann,
und
Y - Pyridyl bedeutet, oder
- für eine Gruppe der Formel $-NR^9R^{10}$ steht,
worin
$R^9$ und $R^{10}$ gleich oder verschieden sind und
- für Wasserstoff oder geradkettiges oder verzweigtes Alkyl oder Alkenyl mit jeweils bis zu 8 Kohlenstoffatomen stehen, das substituiert sein kann durch Fluor, Chlor, Hydroxy, $C_1$-$C_4$-Alkoxy, $C_1$-$C_4$-Alkylthio, $C_1$-$C_4$-Alkylcarbonyl, Carboxy oder $C_1$-$C_6$-Alkoxycarbonyl, oder durch Phenyl, welches seinerseits durch Trifluormethyl $C_1$-$C_2$-Alkyl, Fluor, Chlor oder $C_1$-$C_2$-Alkoxy substituiert sein kann, oder

- für Cycloalkyl mit 3 bis 8 Kohlenstoffatomen stehen, oder
- für Phenyl stehen, das bis zu 2-fach gleich oder verschieden durch Nitro, Cyano, Fluor, Chlor, Brom, $C_1$-$C_4$-Alkyl, $C_1$-$C_4$-Alkoxy, $C_1$-$C_4$-Alkylthio, Trifluormethyl, Trifluormethoxy, Difluormethoxy, Trifluormethylthio, Amino, Alkylamino mit bis zu 4 Kohlenstoffatomen substituiert sein kann, oder
$R^9$ und $R^{10}$ zusammen einen 5- bis 7-gliedrigen gesättigten oder ungesättigten, heterocyclischen Ring bilden, der ein Sauerstoff- oder Schwefelatom oder ein zusätzliches Stickstoffatom enthalten kann, welches gegebenenfalls durch einen Rest $R^{11}$ substituiert ist,
worin
$R^{11}$ - für Wasserstoff steht, oder
- für eine geradkettige oder verzweigte, gesättigte oder ungesättigte Alkylgruppe mit bis zu 4 Kohlenstoffatomen steht, die gegebenenfalls durch Phenyl substituiert ist, welches seinerseits durch Fluor, Chlor, $C_1$-$C_2$-Alkyl, $C_1$-$C_2$-Alkoxy oder $C_1$-$C_2$-Halogenalkyl substituiert sein kann, oder
- für Phenyl steht, das gegebenenfalls durch Fluor, Chlor, Cyano, $C_1$-$C_2$-Alkyl, $C_1$-$C_2$-Alkoxy oder $C_1$-$C_2$-Halogenalkyl substituiert ist
und deren physiologisch unbedenklichen Salze.

9. Verbindungen der allgemeinen Formel (Ia) gemäß Anspruch 8,
in welcher
$R^1$ und $R^2$ gleich oder verschieden sind und
- für Wasserstoff, Cyclopropyl, Cyclopentyl oder Cyclohexyl stehen, oder
- für geradkettiges oder verzweigtes Alkyl oder Alkenyl mit bis zu 8 Kohlenstoffatomen stehen, das gegebenenfalls substituiert ist durch Fluor, Chlor, Hydroxy, $C_1$-$C_4$-Alkoxy, $C_1$-$C_4$-Alkylthio, Carboxy, $C_1$-$C_4$-Alkoxycarbonyl oder durch Phenyl, das seinerseits durch Chlor, Fluor, Methyl oder Methoxy substituiert sein kann, oder
- für Phenyl stehen, das substituiert sein kann durch Fluor, Chlor, Methyl, Ethyl, Methoxy, Trifluormethyl, Trifluormethoxy,
$R^3$ und $R^4$ gleich oder verschieden sind und
- für Methyl stehen
und
X - für einen geradkettigen, verzweigten oder cyclischen, gesättigten oder ungesättigten Kohlenwasserstoffrest mit bis zu 8 Kohlenstoffatomen steht, der gegebenenfalls durch ein Sauerstoff-, Schwefel-oder Stickstoffatom, oder durch einen Rest N-$R^8$ unterbrochen ist,
worin
$R^8$ - Wasserstoff bedeutet, oder
- für Methyl, Ethyl, Benzyl, Phenethyl oder Phenyl steht, das gegebenenfalls durch Fluor, Chlor, Methyl, Methoxy, Trifluormethyl oder Trifluormethoxy substituiert ist,
Y - Pyridyl steht, oder
- für eine Gruppe der Formel -N$R^9R^{10}$ steht,
worin
$R^9$ und $R^{10}$ gleich oder verschieden sind und
- für Wasserstoff oder geradkettiges oder verzweigtes Alkyl oder Alkenyl mit bis zu 4 Kohlenstoffatomen stehen, das substituiert sein kann durch Fluor, Chlor, Hydroxy, $C_1$-$C_4$-Alkoxycarbonyl oder durch Phenyl, das seinerseits durch Fluor, Chlor, Trifluormethyl, Methyl oder Methoxy substituiert sein kann, oder
- für Cyclopropyl, Cyclopentyl oder Cyclohexyl stehen, oder
- für Phenyl stehen, das bis zu 2-fach gleich oder verschieden durch Fluor, Chlor, Methyl, Ethyl, Methoxy, Ethoxy, Methylthio, Trifluormethyl, Trifluormethoxy, Difluormethoxy, Amino, $C_1$-$C_2$-Alkylamino oder $C_1$-$C_2$-Dialkylamino substituiert sein kann, oder
$R^9$ und $R^{10}$ gemeinsam einen 5- bis 7-gliedrigen gesättigten Ring bilden, der ein Sauerstoff-, Schwefel- oder ein zusätzliches Stickstoffatom enthalten kann, das gegebenenfalls durch einen Rest $R^{11}$ substituiert ist,
worin
$R^{11}$ für Wasserstoff, $C_1$-$C_4$-Alkyl, Benzyl oder Phenethyl steht, oder
für Phenyl steht, das durch Fluor, Chlor, Methyl oder Methoxy substituiert sein kann
und deren physiologisch unbedenklichen Salze.

10. Verbindungen der allegemeinen Formel Ia gemäß Anspruch 8, in welcher
$R^1$ und $R^2$ gleich oder verschieden sind und jeweils für Wasserstoff, Alkyl mit 1-4 C-Atomen, oder für Cyclopropyl oder Cyclopentyl stehen,
$R^3$ und $R^4$ für Methyl stehen,
X für eine geradkettige oder verzweigte Alkylkette mit bis zu 4 Kohlenstoffatomen steht, und

Y für Pyridyl oder Morpholino steht oder für eine Gruppe der Formel $-NR^9R^{10}$ steht, worin $R^9$ und $R^{10}$ gleich oder verschieden sind und jeweils Wasserstoff, Methyl Ethyl oder Benzyl bedeuten,
und ihre physiologisch unbedenklichen Salze.

11. Verfahren zur Herstellung von Verbindungen der allgemeinen Formel (Ia)

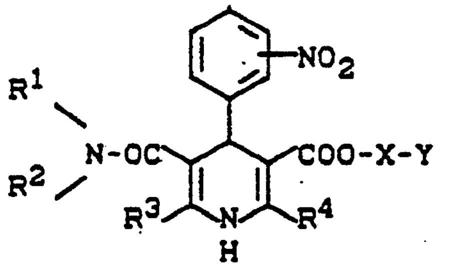

(Ia)

in welcher
$R^1$ und $R^2$ gleich oder verschieden sind und
- Wasserstoff bedeuten, oder
- für Cycloalkyl mit 3 bis 8 Kohlenstoffatomen stehen, oder
- für geradkettiges oder verzweigtes Alkyl oder Alkenyl, mit jeweils bis zu 12 Kohlenstoffatomen stehen, die gegebenenfalls substituiert sind durch Halogen, Hydroxy, Alkoxy mit bis zu 8 Kohlenstoffatomen, Alkylthio mit bis zu 8 Kohlenstoffatomen, Alkylcarbonyl mit bis zu 8 Kohlenstoffatomen, Carboxy oder Alkoxycarbonyl mit bis zu 8 Kohlenstoffatomen oder durch Phenyl, das seinerseits durch Halogen, Nitro, Cyano, Trifluormethyl, Trifluormethoxy, Alkyl mit bis zu 4 Kohlenstoffatomen oder Alkoxy mit bis zu 4 Kohlenstoffatomen oder durch eine Gruppe $-NR^5R^6$ substituiert sein kann,
worin
$R^5$ und $R^6$ gleich oder verschieden sind und Wasserstoff, Alkyl mit bis zu 8 Kohlenstoffatomen bedeuten,
- für Phenyl stehen, das bis zu 3-fach gleich oder verschieden durch Nitro, Cyano, Halogen, Alkyl mit bis zu 6 Kohlenstoffatomen, Alkoxy mit bis zu 6 Kohlenstoffatomen, Alkylthio mit bis zu 6 Kohlenstoffatomen, Trifluormethyl, Trifluormethoxy, Difluormethoxy, Trifluormethylthio, Amino, Alkylamino mit bis zu 8 Kohlenstoffatomen, Dialkylamino mit bis zu 8 Kohlenstoffatomen je Alkylgruppe, Acetylamino oder Benzoylamino substituiert sein kann,
oder
$R^1$ und $R^2$ gemeinsam einen 5- bis 7-gliedrigen gesättigten oder ungesättigten, heterocyclischen Ring bilden, der als zusätzliches Heteroatom ein Sauerstoffatom, ein Schwefelatom, ein Stickstoffatom, oder einen Rest $N-R^7$ enthalten kann,
worin
$R^7$ - Wasserstoff, $C_1$-$C_6$-Alkyl, Aryl oder Aralkyl bedeuten kann
und
$R^3$ und $R^4$ gleich oder verschieden sind und
- für geradkettiges, verzweigtes oder cyclisches Alkyl mit bis zu 8 Kohlenstoffatomen stehen, das gegebenenfalls durch Hydroxy, Cyano, Phenyl oder Halogen substituiert ist
und
X - für einen geradkettigen, verzweigten oder cyclischen, gesättigten oder ungesättigten Kohlenwasserstoffrest mit bis zu 12 Kohlenstoffatomen steht, der gegebenenfalls durch ein Sauerstoff-, Schwefel- oder Stickstoffatom, oder eine Gruppe $N-R^8$ unterbrochen ist,
worin,
$R^8$ - Wasserstoff bedeutet, oder
- für Alkyl mit bis zu 6 Kohlenstoffatomen steht, das gegebenenfalls substituiert sein kann durch Halogen, Hydroxy, Acetoxy, Carboxy, $C_1$-$C_8$-Alkoxycarbonyl oder Phenyl, das seinerseits substituiert sein kann durch Halogen, $C_1$-$C_6$-Alkyl, $C_1$-$C_6$-Alkoxy, Halogenmethyl, Halogenmethoxy oder Cyano,
und
Y - Pyridyl bedeutet, oder
- für eine Gruppe der Formel $-NR^9R^{10}$ steht,
worin
$R^9$ und $R^{10}$ gleich oder verschieden sind und
- für geradkettiges oder verzweigtes Alkyl, Alkenyl oder Alkinyl mit jeweils bis zu 12 Kohlenstoffatomen

stehen, das substituiert sein kann durch Halogen, Hydroxy, Alkoxy mit bis zu 8 Kohlenstoffatomen, Cyano, Trifluormethyl, Alkylthio mit bis zu 8 Kohlenstoffatomen, Alkylcarbonyl mit bis zu 8 Kohlenstoffatomen, Alkoxycarbonyl mit bis zu 8 Kohlenstoffatomen oder durch Phenyl, welches seinerseits durch Nitro, Phenyl, Cyano, Trifluormethyl, Trifluormethoxy, Alkyl mit bis zu 4 Kohlenstoffatomen, Halogen, oder Alkoxy mit bis zu 4 Kohlenstoffatomen substituiert sein kann, oder

- für Cycloalkyl mit 3 bis 8 Kohlenstoffatomen stehen, oder

- für Aryl mit 6 bis 10 Kohlenstoffatomen stehen, das bis zu 3-fach gleich oder verschieden durch Nitro, Cyano, Halogen, Alkyl mit bis zu 6 Kohlenstoffatomen, Alkoxy mit bis zu 6 Kohlenstoffatomen, Alkylthio mit bis zu 6 Kohlenstoffatomen, Carbamoyl, Dialkylcarbamoyl mit jeweils bis zu 6 Kohlenstoffatomen je Alkylgruppe, Trifluormethyl, Trifluormethoxy, Difluormethoxy, Trifluormethylthio, Amino, Alkylamino mit bis zu 8 Kohlenstoffatomen, Dialkylamino mit jeweils bis zu 8 Kohlenstoffatomen je Alkylgruppe substituiert sein kann, oder

$R^9$ und $R^{10}$ zusammen einen 5- bis 7-gliedrigen, gesättigten oder ungesättigten, heterocyclischen Ring bilden, der ein Sauerstoffatom, ein Schwefelatom oder ein zusätzliches Stickstoffatom enthalten kann, das gegebenenfalls durch einen Rest $R^{11}$ substituiert ist,

worin

$R^{11}$ - für Wasserstoff steht, oder

- für eine geradkettige oder verzweigte, gesättigte oder ungesättigte Alkylengruppe mit bis zu 10 Kohlenstoffatomen steht, die gegebenenfalls durch Phenyl substituiert ist, welches seinerseits durch Halogen, $C_1$-$C_4$-Alkyl, $C_1$-$C_4$-Alkoxy, Nitro oder Halogenalkyl mit bis zu 4 Kohlenstoffatomen substituiert sein kann, oder

- für Phenyl steht, das gegebenenfalls durch Halogen, Cyano, Nitro, Alkyl mit bis zu 4 Kohlenstoffatomen oder Halogenalkyl mit bis zu 4 Kohlenstoffatomen substituiert ist,

und deren physiologisch unbedenklichen Salze,

dadurch gekennzeichnet, daß man

[A] Aldehyde der allgemeinen Formel (II)

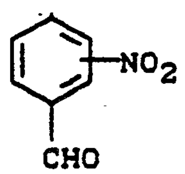

(II)

und $\beta$-Ketocarbonsäureester der allgemeinen Formel (III)

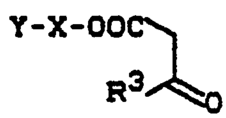

(III)

in welcher

$R^3$, X und Y die oben angegebene Bedeutung haben,

gegebenenfalls nach Isolierung der hieraus entstehenden Ylidenverbindung der allgemeinen Formel (IV)

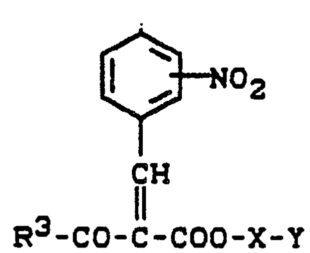

(IV)

in welcher

$R^3$, X und Y die oben angegebene Bedeutung haben,

mit $\beta$-Ketocarbonsäureamiden der allgemeinen Formel (V)

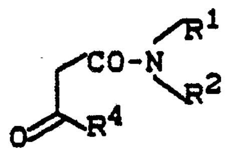

(V)

in welcher
$R^1$, $R^2$ und $R^4$ die oben angebene Bedeutung haben
und Ammoniak bzw. direkt mit den hieraus hergestellten $\beta$-Aminocrotonsäureamiden der allgemeinen Formel (VI)

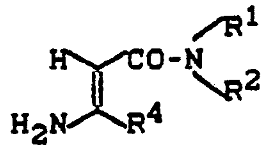

(VI)

in welcher
$R^1$, $R^2$ und $R^4$ die oben angegebene Bedeutung haben,
umsetzt,
oder indem man
[B] Aldehyde der allgemeinen Formel (II) und $\beta$-Ketocarbonsäureamiden der allgemeinen Formel (V) bzw. deren Ylidenverbindungen der allgemeinen Formel (VII)

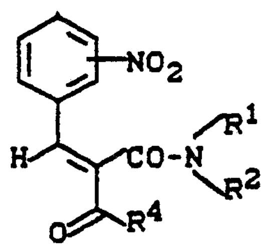

(VII)

in welcher
$R^1$, $R^2$ und $R^4$ die oben angebene Bedeutung haben,
mit $\beta$-Ketocarbonsäureestern der allgemeinen Formel (III) und Ammoniak bzw. direkt mit den hieraus hergestellten Aminocrotonsäureestern der allgemeinen Formel (VIII)

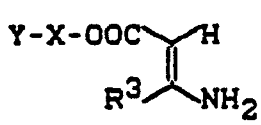

(VIII)

in welcher
$R^3$, X und Y die oben angegebene Bedeutung haben,
umsetzt,
oder man erhält Verbindungen der allgemeinen Formel (I),
in welcher
$R^1$, $R^2$, $R^3$, $R^4$ und X die oben angegebene Bedeutung haben,
Y für eine Gruppe der Formel $NR^9R^{10}$ steht und $R^9$ und/oder $R^{10}$ reaktionsfähigen Wasserstoff bedeuten,
zweckmäßigerweise indem man
[C] Benzylidenverbindungen der allgemeinen Formel (VII) zunächst mit Verbindungen der allgemeinen Formel (VIIIa)

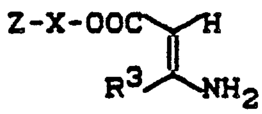

(VIIIa)

in welcher
$R^3$ und X die angegebene Bedeutung haben,
Z - für eine acylierte Aminoschutzgruppe, bevorzugt für tert.-Butoxycarbonylamino oder Phthalimid steht,
zu Verbindungen der allgemeinen Formel (IX)

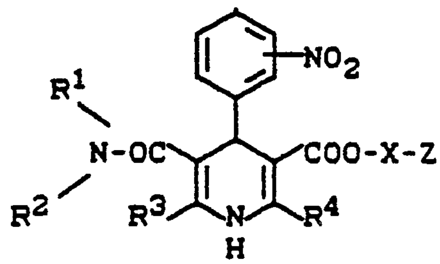

(IX)

in welcher
$R^1$, $R^2$, $R^3$, $R^4$ und X die oben angegebene Bedeutung haben,
und
Z - für die Gruppe

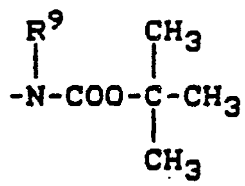

oder

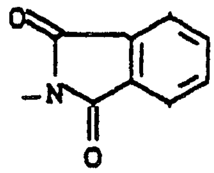

steht,

in einem weiteren Schritt nach bekannten Methoden deblockiert und die erhaltenen Zwischenverbindungen der Formel (IXa),

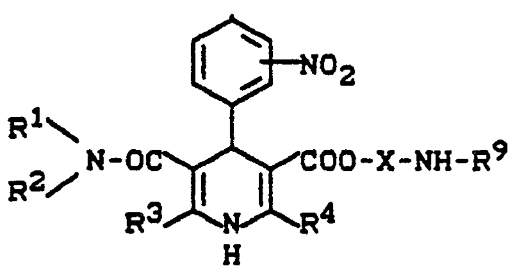

(IXa)

in welcher
$R^1$, $R^2$, $R^3$, $R^4$, X und $R^9$ die oben angegebene Bedeutung haben,
zu Verbindungen der allgemeinen Formel (I),
in welcher
Y - für die Gruppe $NR^9R^{10}$ steht, worin $R^9$ und $R^{10}$ die oben angebene Bedeutung haben, mit der Maßgabe, daß $R^9$ und/oder $R^{10}$ verschieden von Wasserstoff sind,
weiter umsetzt,
oder indem man
[D] Dihydropyridinmonocarbonsäuren der allgemeinen Formel (Xa) oder (Xb)

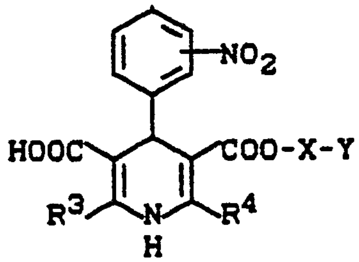

(Xa)

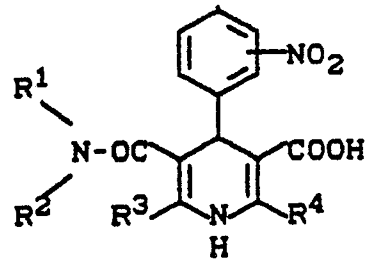

(Xb)

in welchen
$R^1$, $R^2$, $R^3$, $R^4$, X und Y die oben angegebene Bedeutung haben,
gegebenenfalls über reaktive Säurederivate mit Aminen der allgemeinen Formel (XIa)

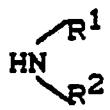

(XIa)

in welcher
$R^1$ und $R^2$ die oben angegebene Bedeutung haben,
bzw. mit Verbindungen der Formel (XIb)
HO-X-Y (XIb)
in welcher
X und Y die oben angegebene Bedeutung haben,
umsetzt, wobei jeweils nur (Xa) mit (XIa) und (Xb) mit (XIb) umgesetzt wird.

12. Verbindungen der allgemeinen Formeln (IX und IXa)

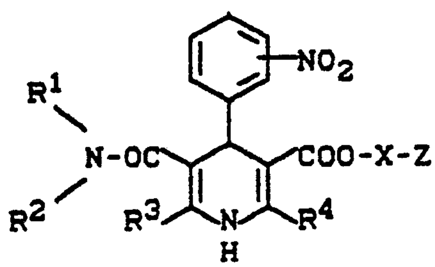

(IX)

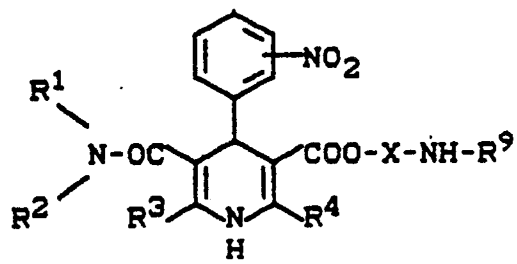

(IXa)

in welchen
$R^1$, $R^2$, $R^3$, $R^4$, X und $R^9$ die im Anspruch 1 angegebene Bedeutung haben und
Z für die Gruppe

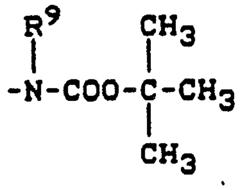

oder

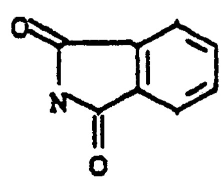

steht.

Europäisches Patentamt

# EUROPÄISCHER RECHERCHENBERICHT

Nummer der Anmeldung

EP 89117497.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.X 5) |
|---|---|---|---|
| D,A | EP - A2/A3 - 0 179 386 (BAYER) * Ansprüche 1,4; Seite 16, Zeilen 13-18 * -- | 1,7, 11,12 | C 07 D 211/90<br>C 07 D 401/12<br>A 61 K 31/44 |
| A | EP - A1 - 0 280 239 (SANWA) * Ansprüche 1,4,5 * ---- | 1,4,5, 7,11 | |

| RECHERCHIERTE SACHGEBIETE (Int. Cl.X 5) |
|---|
| C 07 D 211/00<br>C 07 D 401/00 |

Der vorliegende Recherchenbericht wurde für alle Patentansprüche erstellt.

| Recherchenort | Abschlußdatum der Recherche | Prüfer |
|---|---|---|
| WIEN | 19-12-1989 | HOCHHAUSER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument